# EUROPÄISCHE PATENTSCHRIFT

(11) **EP 2 890 914 B1**
(45) Veröffentlichungstag und Bekanntmachung des Hinweises auf die Patenterteilung: **02.10.2019**
(21) Anmeldenummer: 13770836.8
(22) Anmeldetag: 27.08.2013
(51) Int. Cl.: F16F 9/53, A61F 2/50

(54) **DÄMPFER**
DAMPER
AMORTISSEUR

(30) Priorität: 28.08.2012 DE 102012016948
(43) Veröffentlichungstag der Anmeldung: 08.07.2015
(73) Patentinhaber: Inventus Engineering GmbH, 6771 St. Anton i. M. (AT)
(72) Erfinder: BATTLOGG, Stefan, A-6771 St. Anton im Montafon (AT); KIEBER, Michael, A-6780 Schruns (AT); PÖSEL, Jürgen, A-6700 Bludenz (AT)
(74) Vertreter: BSB Patentanwälte Schütte & Engelen Partnerschaft mbB
(86) Internationale Anmeldenummer: PCT/EP2013/002574
(87) Internationale Veröffentlichungsnummer: WO 2014/032795

(56) Entgegenhaltungen:
- EP-A1- 1 908 985
- JP-A- H06 272 732
- US-A- 5 588 510

## Beschreibung

Die vorliegende Erfindung betrifft einen Dämpfer, der zur Dämpfung der Bewegung einer ersten Komponente relativ zu einer zweiten Komponente dient und ein Verfahren. Die erste und die zweite Komponente können mit dem Dämpfer verbunden sein. Insbesondere werden andere Baueinheiten oder Teile mit der ersten und der zweiten Komponente verbunden sein oder werden. Es ist möglich, dass ein solcher Dämpfer wenigstens teilweise und insbesondere praktisch vollständig in einem Rohrsystem vorgesehen ist, bei dem ein Rohr wie z. B. ein Standrohr und ein relativ dazu bewegliches und insbesondere teleskopierbares zweites Rohr wie z. B. ein Tauchrohr als erste und zweite Komponente vorgesehen sind.

Im Stand der Technik sind verschiedene Dämpfer bekannt geworden. Es sind auch Dämpfer mit feldempfindlichen Fluiden bekannt geworden. Besonders eignen sich magnetorheologische Fluide (MRF) als feldempfindliche Fluide für den Einsatz an Dämpfern. Über ein Magnetfeld kann eine wirksame Dämpfung eingestellt werden.

Als Dämpfungsfluid wird bei magnetorheologischen Fluiden meist ein Dämpfungsmedium auf Ölbasis mit fein verteilten ferromagnetischen Partikeln eingesetzt. Das magnetorheologische Fluid tritt zur Dämpfung durch einen oder mehrere Dämpfungsspalte durch, in denen ein Magnetfeld anliegt. Aufgrund der unterschiedlichen Dämpfungskanäle und der verschiedenen Dämpfungsventile sind Dämpfer meist sehr komplex aufgebaut. Ein besonderes Problem bei Dämpfern für muskelbetriebene Baueinheiten, Bauteile, Fahrzeuge und insbesondere Fahrräder oder Prothesen ist das Bauvolumen. Ein weiterer wichtiger Faktor ist das Gewicht, welches insbesondere bei Prothesen und erst recht bei Dämpfern für den Wettbewerb (z. B. Sport, z. B. Paralympics) und für den anspruchsvollen Amateurbereich von besonderer Bedeutung ist. Weiterhin ist es vorteilhaft, wenn wenigstens einzelne Teile der Dämpfer solche Abmessungen aufweisen, dass sie an anderen Dämpfern benutzt werden können. Sicher einzuhalten sind auf jeden Fall die Einbaumaße. Ein weiteres wichtiges Kriterium ist der mögliche Federweg.

Ein besonders wichtiges Kriterium ist die Grundreibung des Dämpfers bzw. das daraus resultierende sogenannte "Ansprechverhalten". Dämpfer mit magnetorheologischer Flüssigkeit (MR-Flüssigkeit) nach dem Stand der Technik, wie z. B. der MagneRide Dämpfer der BWI Group, verfügen über einen Kolbenstangenausgleich mittels Trennkolben und unter Druck stehendem Gas- oder Luftvolumen. Dieser Druck wirkt so auf das MRF-Volumen im Dämpfer. Daraus resultiert eine Ausfahrkraft des Kolbens bzw. der Kolbenstange, da die Kolbenfläche auf der Zugstufenseite um die Kolbenstangenfläche reduziert ist. Zusätzlich wirkt der Druck im Dämpfer auf die Dichtungen bzw. Dichtlippen z. B. der Kolbenstangendichtung, was höhere Reibung zur Folge hat. Die Ausfahrkraft und die höhere Reibung verschlechtern das Ansprechverhalten eines Dämpfers, was sich besonders bei ganz kleinen Bewegungen (Stössen) negativ auswirkt, diese gehen mitunter ungedämpft auf den zu dämpfenden Körper über.

Weiters bleibt das Kolbenstangenausgleichsvolumen, besonders bei Einwirkung von hohen Druckkräften, nachgiebig. Eine komplett unnachgiebige Druckstufe ist mit solchen Dämpfern nicht umsetzbar.

Aus der EP 1 908 985 B1 ist eine magnetorheologische Dämpfereinrichtung mit einem Einwegkreislauf gemäß dem Oberbegriff von Anspruch 1 bekannt geworden.

Es ist die Aufgabe der vorliegenden Erfindung einen einfach aufgebauten Dämpfer zur Verfügung zu stellen, welche wenigstens einige der oben angeführten Anforderungen erfüllt und besonders die Nachteile beim Ansprechverhalten nicht hat und weitere Einstellmöglichkeiten bietet.

Diese Aufgabe wird gelöst durch einen Dämpfer mit den Merkmalen des Anspruchs 1. Bevorzugte Weiterbildungen der Erfindung sind Gegenstand der Unteransprüche. Weitere Vorteile und Merkmale der Erfindung ergeben sich aus dem Ausführungsbeispiel und der allgemeinen Beschreibung.

Der Dämpfer kann insbesondere zur Dämpfung der Bewegung zwischen einer ersten Komponente und einer relativ zu dieser beweglichen zweiten Komponente dienen. Die erste Komponente und die zweite Komponente können ein teleskopierbares Rohrsystem bilden. Ein solches Rohrsystem kann ein Standrohr und ein relativ dazu bewegliches Tauchrohr aufweisen. Die erste Komponente und die zweite Komponente können Teil des Dämpfers sein.

Der Dämpfer verfügt über wenigstens eine Dämpferkammer und ein steuerbares Drosselventil. Die Dämpferkammer ist durch einen beweglichen und mit einer Kolbenstange verbundenen Kolben in eine erste Kammer und in eine zweite Kammer geteilt. Die erste Kammer ist über einen Rückströmkanal und das Drosselventil mit der zweiten Kammer verbunden.

Es ist wenigstens eine elektronische Steuereinrichtung vorgesehen. Dem Drosselventil ist wenigstens eine durch die elektronische Steuereinrichtung gesteuerte Magnetfeldquelle zugeordnet, um ein durch wenigstens einen Dämpfungskanal des Drosselventils strömendes magnetorheologisches Dämpfungsmedium wenigstens teilweise mit einem magnetischen Feld zu beaufschlagen und dabei zu dämpfen. Die Magnetfeldquelle kann als Magneteinrichtung bezeichnet werden und umfasst vorzugsweise wenigstens eine elektrische Spule.

Es ist wenigstens eine Ausgleichskammer mit einem vorgespannten Ausgleichsvolumen vorgesehen. Dieses kann beispielsweise mittels Trennkolben oder Membran realisiert werden. Das Ausgleichsvolumen ist mit dem Drosselventil und der zweiten Kammer verbunden. Das Drosselventil und die Magnetfeldquelle und die Ausgleichskammer sind extern und damit außerhalb der Dämpferkammer angeordnet. Das Drosselventil und die elektrische Spule sind extern und somit außerhalb der ersten Kammer und der zweiten Kammer angeordnet.

Für das magnetorheologische Dämpfungsmedium ist im Wesentlichen ein Einwegkreislauf vorgesehen, in welchem wenigstens zwei Einwegventile vorgesehen sind, sodass sowohl beim Eintauchen der Kolbenstange in die Dämpferkammer hinein als auch beim Ausfahren bzw. Austauchen der Kolbenstange aus der Dämpferkammer heraus das Dämpfungsmedium in der gleichen Umlaufrichtung umströmt. Es ist ein erstes der Einwegventile an dem Kolben angeordnet und erlaubt eine Strömung des Dämpfungsmediums von der zweiten Kammer in die erste Kammer hinein.

Zwischen dem Drosselventil und der zweiten Kammer ist ein zweites Einwegventil angeordnet, welches eine Strömung des Dämpfungsmediums von dem Drosselventil in die zweite Kammer erlaubt.

Weiterhin ist in weiteres Drosselventil mit einer weiteren Magnetfeldquelle vorgesehen. Das weitere Drosselventil ist in Reihe zu dem Drosselventil geschaltet.

Der erfindungsgemäße Dämpfer hat viele Vorteile. Ein erheblicher Vorteil der erfindungsgemäßen Dämpfer besteht in dem einfachen Aufbau, der sich durch den Einwegkreislauf des MRF ergibt. Das magnetorheologische Dämpfungsmedium strömt beim Einfedern (Druckstufe), wenn der Kolben weiter in die Dämpferkammer eintaucht, von der zweiten Kammer durch das Einwegventil in dem Kolben in die erste Kammer hinein. Durch den Rückströmkanal gelangt das Dämpfungsmedium über das Drosselventil gegebenenfalls wieder in die zweite Kammer hinein.

Das Drosselventil ist vorzugsweise über einen Verbindungskanal mit der zweiten Kammer verbunden. An dem Verbindungskanal ist vorzugsweise das zweite Einwegventil vorgesehen.

Die Einwegventile können nicht nur außen an den jeweiligen Körpern vorgesehen sein, sondern auch in einem Abstand davon angeordnet sein, sofern sie unmittelbar damit verbunden sind. Der Begriff "an" umfasst im Sinne der vorliegenden Erfindung auch den Begriff "in", sodass die Einwegventile auch in dem Kolben bzw. in oder an einem Verbindungskanal zwischen dem Drosselventil und der zweiten Kammer vorgesehen sein können.

Vorzugsweise ist die Magnetfeldquelle insgesamt außerhalb der ersten und zweiten Kammer und insbesondere auch außerhalb der ganzen Dämpferkammer, des Kolbens und der Kolbenstange angeordnet. Die Magnetfeldquelle wird vorzugsweise immer von derselben Seite aus angeströmt. Das magnetorheologische Dämpfungsmedium strömt in dem Kolben/Zylinderraum nur in eine Richtung innerhalb des Einwegkreislaufes.

Besonders vorteilhaft ist auch, dass das magnetorheologische Dämpfungsmedium aufgrund des Einwegkreislaufs immer durchmischt wird.

Das magnetorheologische Dämpfungsmedium kann wenigstens ein magnetorheologisches Fluid umfassen. Insbesondere ist das Dämpfungsmedium als magnetorheologisches Fluid (MRF) ausgebildet. Das Drosselventil ist steuerbar ausgeführt und umfasst wenigstens eine Magnetfeldquelle bzw. Magneteinrichtung als Felderzeugungseinrichtung zur Erzeugung eines Magnetfeldes in wenigstens einem Dämpfungskanal des Drosselventils.

Besonders bevorzugt ist das Drosselventil axial benachbart zu der Dämpferkammer angeordnet. Es ist auch besonders bevorzugt, dass die Ausgleichskammer axial beabstandet von dem Kolben angeordnet ist. Insbesondere ist die Ausgleichskammer axial außerhalb und vorzugsweise axial benachbart zu der Dämpferkammer und vorzugsweise axial neben der zweiten Kammer angeordnet.

Das Ausgleichsvolumen ist mit dem Drosselventil und der zweiten Kammer verbunden. Das Ausgleichsvolumen ist insbesondere über den Verbindungskanal mit der zweiten Kammer verbunden. Durch insbesondere das erste Einwegventil ist es möglich, die Ausgleichskammer und das Ausgleichsvolumen immer in den Niederdruckbereich, d. h. hinter das Drosselventil zu schalten. Dadurch muss das Ausgleichsvolumen nur mit geringem Druck vorgespannt werden und es wird trotzdem auch bei hoher Dämpfung ein steifes System erhalten, welches nicht in das Ausgleichsvolumen arbeitet. Zudem bewirkt das Ausgleichsvolumen so nur eine geringe Ausfahrkraft auf den Kolben, was das Ansprechverhalten wesentlich verbessert.

Eine solche Ausgestaltung hat erhebliche Vorteile. Sowohl beim Einfedern (Druckstufe) als auch beim Ausfedern (Zugstufe) fließt wenigstens ein Teil des Dämpfungsmediums durch das Drosselventil durch. Beim Einfedern tritt die Kolbenstange weiter in die Dämpferkammer ein, sodass das Dämpfungsmedium durch das erste Einwegventil in dem Kolben in die erste Dämpferkammer übertreten muss. Der Weg aus der zweiten Dämpferkammer nach außen über das zweite Einwegventil ist verschlossen, da dieses nur das Einströmen des Dämpfungsmediums durch den Verbindungskanal in die zweite Dämpferkammer hinein erlaubt. In Gegenrichtung sperrt das Einwegventil.

Beim Eintauchen verdrängt der Kolben ein Volumen, welches proportional zu seiner Querschnittsfläche ist. In der ersten Federkammer wird allerdings nur ein Volumen frei, welches proportional zur Querschnittsfläche des Kolbens abzüglich der Querschnittsfläche der Kolbenstange ist. Deshalb muss beim Eintauchen des Kolbens ein Teil des Dämpfungsmediums durch den Rückströmkanal zu dem Drosselventil strömen. Dort findet eine entsprechende Drosselung statt. Dieser Teil des Dämpfungsmediums tritt anschließend in die Ausgleichskammer ein.

Beim Ausfedern tritt die Kolbenstange aus der Dämpferkammer aus und es muss in der zweiten Dämpferkammer ein Volumen des Dämpfungsmediums nachströmen, welches proportional zu der Querschnittsfläche des Dämpferkolbens ist. Da das erste Einwegventil in dem Kolben nur eine Strömung des Dämpfungsfluids von der zweiten Dämpferkammer zu der ersten Dämpferkammer erlaubt und in Gegenrichtung sperrt, muss das Dämpfungsmedium über den Verbindungskanal durch das nun öffnende zweite Einwegventil in die zweite Dämpferkammer eintreten. Gleichzeitig tritt aus der ersten Dämpferkammer das verdrängte Dämpfungsmedium aus, welches über den Rückströmkanal zu der Drosseleinrichtung gelangt. Da in der zweiten Dämpferkammer mehr Volumen benötigt als in der ersten Dämpferkammer verdrängt wird, muss ein dem Querschnitt der Kolbenstangen entsprechender Anteil aus der Ausgleichskammer abgegeben werden. Damit liegen sowohl in dem Rückströmkanal als auch in dem Verbindungskanal sowohl beim Einfedern als auch beim Ausfedern Strömungen in der gleichen Umlaufrichtung vor.

Das ist vorteilhaft, da über ein einziges Drosselventil sowohl die Zugstufe als auch die Druckstufe gedämpft werden kann. Das erleichtert den Aufbau eines solchen Dämpfers erheblich. Es kann auch Gewicht eingespart werden und das stetige Strömen führt zum guten Durchmischen der magnetorheologischen Flüssigkeit.

Ein Vorteil einer solchen Lösung ist, dass der Volumenausgleich immer in dem Niederdruckbereich angeordnet ist. Das bedeutet, dass der Volumenausgleich in Strömungsrichtung gesehen immer hinter dem Drosselventil oder den Drosselventilen geschaltet ist. Vorzugsweise ist der Vorspanndruck im Ausgleichsvolumen kleiner als 5 bar. Der Vorspanndruck kann auch nur 2 oder 3 bar betragen.

In besonders bevorzugten Ausgestaltungen ist die Dämpferkammer derart angeordnet und/oder derart verschaltet, dass der Zugstufe mehr effektiv wirksame Kolbenfläche als der Druckstufe zugeordnet ist. Das bedeutet, dass in der Zugstufe bei einem bestimmten Hub mehr Volumen des Dämpfungsmediums durch das Drosselventil durchtritt als bei gleichem Hub in der Druckstufe. Unter dem Begriff "effektiv wirksame Kolbenfläche" wird hier die Relation zu dem durch das Drosselventil durchtretenden Volumenstrom verstanden.

Bei den meisten Konstruktionen ist die effektiv wirksame Kolbenfläche in der Druckstufe größer. Das trifft insbesondere auf die Lösungen zu, bei denen das effektiv wirksame Drosselventil in dem Kolben selbst angeordnet ist.

Oftmals werden aber Dämpfungsverhältnisse gewünscht, die eine größere Dämpfung in der Zugstufe bewirken. Eine solche Lösung kann hier konstruktiv vorteilhaft erreicht werden. Eine Zugstufe mit einer steileren Kennlinie kann einfach ermöglicht werden.

In bevorzugten Ausgestaltungen ist es möglich, dass das Drosselventil über ein erstes Rückschlagventil mit der Ausgleichskammer verbunden ist. Dabei erlaubt das erste Rückschlagventil nur eine Strömung des Dämpfungsmediums von dem Drosselventil in die Ausgleichskammer hinein.

Vorzugsweise ist die Ausgleichskammer über ein zweites Rückschlagventil mit der zweiten Kammer verbunden. Dabei erlaubt das zweite Rückschlagventil nur eine Strömung des Dämpfungsmediums von der Ausgleichskammer in die zweite Kammer hinein.

Besonders bevorzugt ist wenigstens eines der Einwegventile und/oder der Rückschlagventile einstellbar, um einen einstellbaren Strömungswiderstand in der Druckstufe und/oder der Zugstufe zu ermöglichen. Bei einer solchen Ausgestaltung ist es möglich, dass die Ausgleichskammer über zwei separate Ausgleichskanäle mit dem Verbindungskanal verbunden ist. Der eine Ausgleichskanal ist mit dem ersten Rückschlagventil versehen, während der zweite Ausgleichskanal mit dem zweiten Rückschlagventil ausgerüstet ist.

In diesen Ausgestaltungen ist es insbesondere möglich, dass die Rückschlagventile von außen an dem Dämpfer einstellbar sind, um beispielsweise bei mechanischen Rückschlagventilen die Kennlinie zu verändern. Dann ist das Rückschlagventil als einstellbares Drosselventil mit Rückschlagvorrichtung ausgebildet. Wird bei einer solchen Ausgestaltung ein mechanisches Drosselventil eingesetzt, so wird mit dem Drosselventil beispielsweise die Grundkennlinie eingestellt, während über die beiden einstellbaren Rückschlagventile beispielsweise die Grundkennlinie an die gewünschte Kennlinie im Zugstufenfall und die gewünschte Kennlinie im Druckstufenfall angepasst wird.

Wenigstens ein Einwegventil und/oder wenigstens ein Rückschlagventil kann justierbar sein. Beispielsweise kann ein solches Ventil mechanisch justierbar ausgeführt sein, um Grundeinstellungen vorzunehmen.

Es ist insbesondere möglich, dass das Drosselventil steuerbar ausgeführt ist und ein entsprechend gesteuertes Magnetfeld in dem Dämpfungskanal des wenigstens eines Drosselventils erzeugt. Grundsätzlich sind neben magnetorheologischen Fluiden auch elektrorheologische Fluide (ERF) bekannt geworden. Für die vorgesehenen Einsatzzwecke eignet sich ein MRF aber erheblich besser, da ERF hohe Spannungen zur Steuerung benötigen. Ein weiterer Nachteil von ERF ist, dass sich keine dauerhaften Felder induzieren lassen. Bei MRF ist es hingegen möglich, mit Permanentmagneten stromlos bestimmte Drosselzustände einzustellen oder die Remanenz von Materialien auszunutzen. Dabei wird die Magnetfeldstärke eines Permanentmagneten durch beispielsweise einen kurzen magnetischen Impuls dauerhaft eingestellt. Die eingestellte Magnetfeldstärke bleibt auch lange nach dem magnetischen Impuls erhalten, ohne dass weiterhin äußere Energie benötigt wird. Diese Möglichkeiten gibt es bei ERF nicht.

Eine solche Ausgestaltung ist besonders vorteilhaft, da magnetorheologische Dämpfungsmedien schnell auf angelegte Magnetfelder reagieren. Dabei ist es möglich, dass ein Dauermagnet als Felderzeugungseinrichtung eingesetzt wird. Ein solcher Dauermagnet kann beispielsweise mechanisch in seiner Lage verändert werden, um die in dem Dämpfungskanal wirkende Dämpfungskraft verändern zu können. Möglich ist es auch, dass ein Dauermagnet eingesetzt wird, dessen Magnetfeld durch das Magnetfeld einer elektrischen Spule je nach den gerade gewünschten Anforderungen überlagert wird. So kann durch den Dauermagneten eine ständig wirkende Dämpfung eingestellt werden, die beispielsweise bei Bedarf durch das Magnetfeld der elektrischen Spule abgeschwächt oder verstärkt wird.

Möglich ist es auch, dass die Felderzeugungseinrichtung einen hier sogenannten Remanenzmagneten umfasst, dessen Magnetfeldstärke periodisch bei Bedarf oder in unregelmäßigen Abständen durch einen magnetischen Impuls einer zugeordneten elektrischen Spule eingestellt wird. Ein solcher Remanenzmagnet wird durch den magnetischen Impuls von beispielsweise nur einigen Millisekunden Dauer dauerhaft auf eine bestimmte Magnetfeldstärke eingestellt. Wenn die magnetische Magnetfeldstärke des Remanenzmagneten wieder reduziert werden soll, kann dies z. B. über ein zeitlich sich abschwächendes Wechselfeld erfolgen. Eine Lösung für den grundsätzlichen Aufbau eines Drosselventils mit einem Remanenzmagneten kann insbesondere der EP 2 339 203 A2 entnommen werden. Ein bevorzugter Aufbau eines mit Remanenz arbeitenden Ventils orientiert sich vorzugsweise an diesem Dokument.

Mit einem elektrisch einstellbaren Drosselventil und einem magnetorheologisches Fluid wird eine besonders flexible Steuerung der Dämpfungseigenschaften ermöglicht. Unabhängig von mechanischen Einstellmöglichkeiten eröffnet ein solches steuerbares Drosselventil die Möglichkeit der Steuerung in Echtzeit, bei der auf einen Stoß in Echtzeit reagiert wird, noch während der Stoß stärker wird und bevor er sein Maximum erreicht. Das kann hier durch die Reaktionsgeschwindigkeit eines beispielsweise magnetorheologischen Fluides gewährleistet werden, welches innerhalb von einer Millisekunde oder etwas mehr sich entlang der Feldlinien eines Magnetfeldes verketten kann und somit quer dazu den Strömungswiderstand erheblich vergrößern kann.

Erfindungsgemäß weist wenigstens eine Magnetfeldquelle bzw. Magneteinrichtung wenigstens eine insbesondere außerhalb der ersten und der zweiten Kammer angebrachte elektrische Spule auf, bei der eine Spulenachse quer zu der Strömungsrichtung des magnetorheologischen Dämpfungsmediums ausgerichtet ist. Dadurch wird eine besonders hohe Wirksamkeit erzielt. Eine solche elektrische Spule kann als "liegende" elektrische Spule bezeichnet werden. Die elektrische Spule ist insbesondere außerhalb des Kolben-/Zylinderraumes vorgesehen und kann sogar rechtwinklig zu dem Dämpfungskanal in dem Drosselventil angeordnet sein. Vorzugsweise ist die elektrische Spule so angeordnet, dass wenigstens ein erheblich Teil des erzeugten Magnetfeldes auf den Dämpfungskanal einwirkt.

In allen Ausgestaltungen sind vorzugweise wenigstens eine Steuereinrichtung und/oder wenigstens eine Sensoreinrichtung vorgesehen. Mit der Steuereinrichtung kann das steuerbare Drosselventil in Abhängigkeit von Sensorsignalen eingestellt werden. Grundsätzlich können unterschiedlichste Sensoren vorgesehen sein.

Besonders bevorzugt ist wenigstens eine Sensoreinrichtung zur Erkennung wenigstens einer Stellgröße vorgesehen.

Vorzugsweise ist wenigstens eine Sensoreinrichtung dazu vorgesehen, ein Maß für eine Relativgeschwindigkeit zu erfassen.

Insbesondere ist die Sensoreinrichtung dazu vorgesehen, ein Maß für eine Relativgeschwindigkeit des Kolbens zu der Dämpferkammer zu erfassen. Möglich ist es aber auch, dass eine Sensoreinrichtung eine Relativgeschwindigkeit der ersten und der zweiten Komponente zueinander erfasst. Möglich ist es auch, dass eine Relativgeschwindigkeit einer Komponente beispielsweise in einer bevorzugten Richtung (z. B. in vertikaler Richtung) erfasst wird, um daraus auf die aktuelle Belastung rückschließen zu können. Möglich ist auch die Erfassung der Beschleunigung(en) durch einen oder mehrere Sensoren. Möglich ist auch die Kombination von verschiedenen Sensortypen.

Besonders bevorzugt ist wenigstens eine Sensoreinrichtung dazu vorgesehen, eine Richtung der Relativbewegung zwischen den Kolben und der Dämpferkammer zu erfassen. Das ist z. B. bei der Verwendung von magnetorheologischen Fluiden von Bedeutung, da ohne Weiteres allein durch das Strömen des Dämpfungsmediums innerhalb des Einwegkreislaufes keine Aussage darüber getroffen werden kann, ob es sich um strömendes Dämpfungsmedium bei einem Einfedervorgang oder einem Ausfedervorgang handelt. Zur Lösung dieses Problems kann in einfachen Fällen wenigstens eine Sensoreinrichtung zur Erfassung der Richtung der Relativbewegung vorgesehen sein, die beispielsweise als Detektor wenigstens ein auslenkbares Federplättchen umfasst, welches vorzugsweise durch entsprechende Vorbelastungseinrichtungen in eine Mittelstellung vorbelastet ist.

Eine solche Sensoreinrichtung kann beispielsweise in der Ausgleichskammer oder an einem Ausgleichskanal vorgesehen sein, der zu der Ausgleichskammer führt. Mit der Auslenkung des beispielsweise als Detektor dienenden Federplättchens kann erfasst werden, ob das Dämpfungsmedium aus der Ausgleichskammer herausströmt oder aber in sie hinein. Dementsprechend kann mit der Auslenkung des Detektors bestimmt werden, ob ein Einfeder- oder ein Ausfedervorgang vorliegt. Der Detektor muss nur in einem Bereich angeordnet werden, der in beiden Fällen von dem Dämpfungsmedium entsprechend durchströmt wird. Möglich ist es auch, dass zwei separate Sensoreinrichtungen vorgesehen sind, die separat das Einfedern und das Ausfedern erfassen.

Möglich ist es aber auch, dass eine Sensoreinrichtung vorhanden ist, die ein Maß für einen Federweg erfasst. Durch die zeitliche Änderung des Federweges kann darauf zurückgeschlossen werden, ob der Dämpfer einfedert oder ausfedert. Möglich ist auch der Einsatz wenigstens eines Beschleunigungssensors, aus dessen oder deren Daten auf ein Einfedern oder ein Ausfedern geschlossen werden kann.

Möglich ist es aber auch, dass ein Drucksensor vorhanden ist, der den Differenzdruck zwischen der Ausgleichskammer und einem über eine Blende angrenzenden Referenzvolumen misst. Der Differenzdruck über die Blendenöffnung ist dann proportional zur Ein- oder Ausfedergeschwindigkeit.

In allen Ausgestaltungen ist es möglich und bevorzugt, dass wenigstens eine Endlagendämpfung vorgesehen ist. Eine solche Endlagendämpfung kann die Dämpfung in einem Endbereich beim Einfedern oder Ausfedern entsprechend verstärken, um einen Durchschlag an dem Dämpfer zu verhindern.

Besonders bevorzugt ist im bestimmungsgemäßen Gebrauch die erste Kammer auf der einen Seite der zweiten Kammer angeordnet. Vorzugsweise ist das Drosselventil dann auf der anderen Seite der Dämpferkammer angeordnet. Die Ausgleichskammer ist wiederum vorzugsweise benachbart zu dem Drosselventil angeordnet. Besonders bevorzugt ist im bestimmungsgemäßen Gebrauch die erste Kammer unterhalb der zweiten Kammer angeordnet. Vorzugsweise ist das Drosselventil oberhalb der Dämpferkammer angeordnet. Besonders bevorzugt ist die Ausgleichskammer oberhalb des Drosselventils vorgesehen. Die Ausgleichskammer kann auch unterhalb oder seitlich des Drosselventils vorgesehen sein, was die Strömungswege reduziert.

Möglich und bevorzugt ist auch ein umgekehrter Aufbau bzw. eine umgekehrte oder liegende Verwendung. Dann ist im bestimmungsgemäßen Gebrauch die erste Kammer oberhalb (oder z. B. links) der zweiten Kammer angeordnet. Vorzugsweise ist das Drosselventil unterhalb (rechts) der Dämpferkammer angeordnet. Besonders bevorzugt ist die Ausgleichskammer unterhalb (rechts) des Drosselventils vorgesehen. Die Ausgleichskammer kann auch oberhalb (links) oder seitlich des Drosselventils vorgesehen sein, was die Strömungswege reduziert.

Bei solchen Ausgestaltungen wird ein besonders einfacher Aufbau ermöglicht. Gleichzeitig kann das Nachfüllen der Ausgleichskammer mit beispielsweise Druckluft einfach erfolgen. Das oben angeordnete Drosselventil oder die darüber angeordnete Ausgleichskammer ermöglicht auch das einfache Nachfüllen oder das einfache Austauschen vom Dämpfungsmedium. Auch die Wärmeabfuhr kann einfach erfolgen.

Ein erheblicher Vorteil einer solchen Ausgestaltung ist auch, dass der um einen Dämpfer vorhandene Platz vorteilhaft ausgenutzt werden kann.

In bestimmten bevorzugten Weiterbildungen sind wenigstens die Dämpferkammer und das Drosselventil in einem Rohrsystem angeordnet sind. Dabei entspricht die erste Komponente einem ersten Rohr und die zweite Komponente einem zweiten Rohr, die insbesondere teleskopierbar sind.

Während bei derartigen Rohrsystemen meist nur ein geringer Durchmesser zur Verfügung steht, kann innerhalb der Rohre deren Länge ausgenutzt werden.

Besonders bevorzugt sind Einsatzeinrichtungen oder wenigstens eine Einsatzeinrichtung zwischen dem Rohrsystem und der Dämpferkammer vorgesehen. Die Einsatzeinrichtung ist dabei derart gestaltet, dass der Rückströmkanal wenigstens abschnittsweise an der Einsatzeinrichtung vorgesehen ist. Vorzugsweise begrenzt die Einsatzeinrichtung den Strömungsquerschnitt des Rückströmkanals. Durch die Einsatzeinrichtung kann der Strömungsquerschnitt des Rückströmkanals erheblich reduziert werden. Damit kann das Gesamtgewicht des Dämpfers deutlich reduziert werden, was bei hohen Anforderungen von erheblichem Vorteil ist.

Insbesondere ist eine größte Länge, Erstreckung oder ein größter Durchmesser eines Strömungsquerschnittes des Rückströmkanals an der Einsatzeinrichtung kleiner als ein Durchmesser des Rohrsystems. Insbesondere ist eine größte Länge oder eine größte Erstreckung des Strömungsquerschnittes an der Einsatzeinrichtung quer zur Strömungsrichtung kleiner als ein Radius und besonders bevorzugt kleiner als ein halber Radius des Rohrsystems. Insbesondere beziehen sich die Abmessungen des Rohrsystems hier auf den Außendurchmesser und besonders bevorzugt den Innendurchmesser des inneren Rohres.

Es ist möglich, dass der Zwischenraum zwischen der äußeren Umwandung der Dämpferkammer und der Innenwandung eines Rohres vollständig als Strömungskanal genutzt wird. Bei einer solchen Ausgestaltung würde hier der gesamte Zwischenraum zwischen der Außenwandung der Dämpferkammer und der Innenwandung des inneren Rohres mit dem Dämpfungsmedium angefüllt. Durch das erhebliche Volumen dieses Zwischenraums wäre dort eine erhebliche Menge an Dämpfungsmedium vorhanden, die das Gesamtgewicht des Dämpfers ganz erheblich erhöhen würde. Eine Lösung zur Reduzierung des Gewichts könnte darin bestehen, den Zwischenraum zu verkleinern, in dem beispielsweise der Innenraum bzw. der Innendurchmesser des inneren Rohres verkleinert wird. Dadurch würde ein kleinerer Zwischenraum entstehen, sodass dort eine geringere Masse an Dämpfungsmedium vorhanden wäre. Doch eine solche Lösung hätte den Nachteil, dass die Kompatibilität des Dämpfers mit üblicherweise verwendeten Abmessungen nicht mehr gegeben wäre. Es könnten nicht Rohre eingesetzt werden, wie sie derzeit üblich sind. Das würde die Konstruktion eines solchen Dämpfers erheblich aufwendiger gestalten.

Alternativ dazu könnte auch der Außendurchmesser der Dämpferkammer vergrößert werden, um einen kleineren Spalt in dem Zwischenraum zu ermöglichen. Auch bei dieser Lösung würde sich eine geringere Masse an Dämpfungsmedium in dem Zwischenraum sammeln, sodass das Gewicht reduzierbar wäre. Nachteilig bei dieser Lösung ist allerdings, dass die Wandreibung bei der Strömung des Dämpfungsmediums ganz erheblich zunehmen würde. Dadurch wäre es schwierig, die geforderten Dämpfungseigenschaften einzustellen, da durch den hohen Strömungswiderstand im Zwischenraum niedrige Dämpfungswerte praktisch nicht mehr einstellbar wären.

Auch bei einer Verringerung des Innendurchmessers des inneren Rohres würde sich übrigens eine entsprechende Erhöhung des Strömungswiderstandes des Dämpfungsmediums ergeben.

Das bedeutet, dass sowohl eine Verringerung der Durchmesser des inneren Rohres als erster Komponente als auch eine Vergrößerung der Dämpferkammer keine zufriedenstellende Lösung bietet. Die erstaunliche Lösung ist nun, wenigstens eine Einsatzeinrichtung in dem Zwischenraum zu positionieren, die einen genau definierten Rückströmkanal begrenzt. Der Rückströmkanal an der Einsatzeinrichtung weist dabei vorzugsweise eine geringe Umfangsfläche im Vergleich zu seinem Querschnitt auf. Dadurch wird die Wandreibung an dem Rückströmkanal reduziert. Die im Vergleich zur Umfangsfläche große Querschnittsfläche erlaubt hohe Strömungsgeschwindigkeiten des Dämpfungsmediums, ohne den Strömungswiderstand unzulässig zu erhöhen.

Vorzugsweise wird zusätzlich die Einsatzeinrichtung aus einem solchen Material und derart konstruiert, dass sich eine mittlere Dichte der Einsatzeinrichtung zwischen dem Rohrsystem und der Dämpferkammer ergibt, die kleiner als eine mittlere Dichte des Dämpfungsmediums ist. Durch eine solche Maßnahme wird sichergestellt, dass Gewicht eingespart werden kann.

In bevorzugten Ausgestaltungen ist die mittlere Dichte der Einsatzeinrichtung geringer als halb so groß wie die mittlere Dichte des Dämpfungsmediums oder doch wenigstens niedriger als drei Viertel der Dichte des Dämpfungsmediums. Dadurch kann eine erhebliche Gewichtsreduktion des Dämpfers erfolgen, während gleichzeitig sowohl hohe Dämpfungsraten als auch geringe Dämpfungsraten einstellbar sind. Durch abgeschlossene Hohlräume in der Einsatzeinrichtung oder besonders leichte Materialien kann das Gewicht erheblich reduziert werden.

In allen Ausgestaltungen ist es bevorzugt, dass an dem Drosselventil die Strömung des magnetorheologischen Dämpfungsmediums mittels der Magnetfeldquelle bzw. Magneteinrichtung variiert und deren Schaltzustand dann stromlos gehalten werden kann.

In bevorzugten Ausgestaltungen ist wenigstens ein weiteres Drosselventil als Absenkventil mit einer weiteren Felderzeugungseinrichtung vorgesehen. Das weitere Drosselventil kann beispielsweise dafür vorgesehen sein, den Dämpfer bei Prothesen beim Sitzen abzusenken (abzuwinkeln) oder abgesenkt (abgewinkelt) zu halten.

Das weitere Drosselventil als z. B. Absenkventil kann ebenso wie das oben beschriebene Drosselventil eine elektrische Spule als Felderzeugungseinrichtung umfassen. Es ist auch möglich, dass das weitere Drosselventil ebenso wie das oben beschriebene Drosselventil wenigstens einen Remanenzmagnet und/oder wenigstens einen Permanentmagnet als Magneteinrichtung bzw. Felderzeugungseinrichtung aufweist. In allen Ausgestaltungen ist das weitere Drosselventil in Reihe zu dem Drosselventil geschaltet.

Wird beispielsweise eine elektrische Spule eingesetzt, so wird mit dieser Magneteinrichtung nur dann ein entsprechendes Magnetfeld erzeugt, wenn die Absenkung vom Dämpfer beim Sitzen beispielsweise erwünscht ist und wenn sich der Dämpfer im Ausfederungszustand befindet. Dann sorgt die erhöhte Dämpfung für eine zuverlässige Positionierung des Dämpfers im abgesenkten Zustand. Gleichzeitig kann noch eine Dämpfung von Stößen erfolgen. Später kann das Absenkventil wieder ausgeschaltet werden, sodass der Dämpfer im normalen Betrieb (z. B. Gehen) schnell wieder ihre normale ausgefederte Position erreicht.

Wenn beispielsweise nur ein Permanentmagnet als Magnetfeldquelle für das Absenkventil gewählt wird, so wird dieser regelmäßig sowohl bei der Zugstufendämpfung als auch bei der Druckstufendämpfung wirken. Dadurch ergibt sich nach einer erstmaligen Einfederung ein stärkeres Dämpfungsverhalten vom Dämpfer.

Es ist möglich, dass beispielsweise ein Permanentmagnet mechanisch zwischen einer Normalstellung und einer weiteren Stellung wie beispielsweise einer Absenkstellung beweglich ist. Der Permanentmagnet kann beispielsweise an einer drehbaren Einrichtung vorgesehen sein, die das Rohrsystem außen umgibt. Mit einem Einstellhebel kann der Permanentmagnet in die gewünschte Winkelstellung gebracht werden, wo er berührungslos durch das Rohrsystem hindurch auf das weitere Drosselventil einwirkt.

In allen Ausgestaltungen ist es bevorzugt, dass wenigstens ein Einwegventil und/oder wenigstens ein Rückschlagventil als Shim-Ventil ausgebildet ist bzw. sind. Ein solches Shim-Ventil kann einen Stapel unterschiedlicher Scheiben aufweisen, die ein nichtlineares Verhalten an dem Rückschlagventil zur Verfügung stellen.

Besonders bevorzugt ist wenigstens ein Einwegventil und/oder wenigstens ein Rückschlagventil justierbar. Das kann beispielsweise von außen erfolgen.

Es ist bevorzugt, dass wenigstens eine einstellbare Ventileinrichtung vorgesehen ist, welche über Remanenzeigenschaften verfügt. Eine einstellbare Ventileinrichtung kann aus einem Ventil oder aus zwei oder mehr in Serie geschalteten Einzelventilen bestehen. Eines der Ventile kann als Sperrventil ausgebildet sein, welches insbesondere rein mechanisch z. B. als Shim-Ventil das Dämpfungsmedium nur in eine Richtung durchlässt. Ein weiteres Ventil bzw. Teilventil kann in das Sperrventil der einstellbaren Ventileinrichtung integriert sein oder benachbart zu dem Sperrventil angeordnet sein. Das weitere Ventil kann auf mechanischer und/oder elektrischer und/oder auf magnetorheologischer Basis arbeiten und durch Erzeugen oder Anlegen eines einstellbaren, vorbestimmten oder festen Magnetfeldes die Strömung durch einen Dämpfungskanal des weiteren Ventils im gewünschten Ausmaß dämpfen. Das weitere Ventil innerhalb der einstellbaren Ventileinrichtung kann auf Basis von Remanenz vorgesehen sein. Dann ist dem weiteren Ventil eine elektrische Spule zur Erzeugung von magnetischen Impulsen zugeordnet, mit welchen ein dauerhaft wirkendes Magnetfeld in einem hart- oder weichmagnetischen Material verändert oder eingestellt wird.

Möglich ist es auch, dass die einstellbare Ventileinrichtung wenigstens einen Permanentmagneten und/oder wenigstens eine elektrische Spule zur Erzeugung bzw. Anlegung eines gewünschten Magnetfeldes aufweist.

Bevorzugt ist es auch, dass wenigstens eine einstellbare Ventileinrichtung ein Remanenzventil umfasst oder aus nur einem ein Remanenzventil besteht, welches auf magnetorheologischer Basis arbeitet und dessen Magnetfeld durch wenigstens einen Impuls einer elektrischen Spule einstellbar ist.

Grundsätzlich ist es bevorzugt, dass wenigstens ein einstellbares Ventil und/oder wenigstens ein Rückschlagventil als einstellbare Ventileinrichtung ausgeführt ist.

Bei dem Dämpfer gemäß der Erfindung kann alles kompakt aufgebaut sein. Alle Komponenten können ineinander geschachtelt und übereinander angeordnet sein. So sind die elektronische Steuereinrichtung (Elektronik) und der Akku vorzugsweise axial gesehen benachbart von dem Ausgleichsvolumen angeordnet, sodass das Ausgleichsvolumen zwischen dem Akku und der Dämpferkammer angeordnet ist.

Ein erfindungsgemäßes Verfahren dient zum Bereitstellen eines Dämpfers mit konstruktiv bedingter niederer Grundreibung und wenigstens einer steifen Druckstufe, die auch bei hoher Dämpfung erhalten bleibt, insbesondere weil sie nicht in das Ausgleichsvolumen arbeitet. Dazu werden die Ventile vorzugsweise so verschaltet, dass das Ausgleichsvolumen in Strömungsrichtung gesehen immer hinter dem Drosselventil angeordnet ist. Insbesondere ist auch die Zugstufe steif ausgeführt, wobei die Steifheit auch bei einer hohen Dämpfung erhalten bleibt. Das wird vorzugsweise realisiert, indem die Ventile insbesondere so verschaltet sind, dass das Ausgleichsvolumen auch im Zugstufenfall in Strömungsrichtung gesehen immer hinter dem Drosselventil angeordnet ist.

Weitere Vorteile und Merkmale der vorliegenden Erfindung ergeben sich aus der Beschreibung der Ausführungsbeispiele, die im Folgenden mit Bezug auf die beiliegenden Figuren erläutert werden.

In den Figuren zeigen:
- Fig. 1: eine Vorderansicht eines erfindungsgemäßen Dämpfers;
- Fig. 2: ein Rohrsystem mit einer Dämpfereinrichtung für den Dämpfer nach Fig. 1 in einer ersten Ausführungsform;
- Fig. 3: ein Rohrsystem mit einer Dämpfereinrichtung für den Dämpfer nach Fig. 1 in einer zweiten Ausführungsform;
- Fig. 4: ein Rohrsystem mit einer Dämpfereinrichtung für den Dämpfer nach Fig. 1 in einer dritten Ausführungsform;
- Fig. 5: einen schematischen Querschnitt durch ein Rohrsystem eines Dämpfers nach den Fig. 2 bis 4;
- Fig. 6: einen schematischen Querschnitt durch ein Drosselventil, wie es in einem vorhergehenden Ausführungsbeispiel eingesetzt werden kann; und
- Fig.7: einen schematischen Querschnitt durch ein Rohrsystem mit einem ein Drosselventil, wie es in einem vorhergehenden Ausführungsbeispiel eingesetzt werden kann.

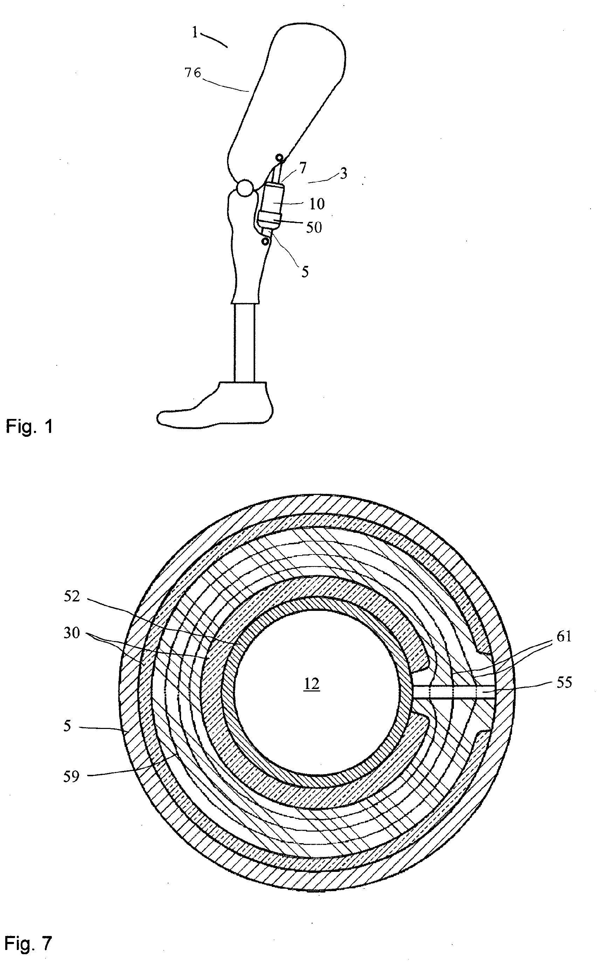

Fig. 1 zeigt als eine mögliche Anwendung dieser Erfindung deren Einsatz als Dämpfer bei einer Beinprothese 76. Bei dieser Anwendung sind eine niedrige Grundreibung, ein Absenken und eine unnachgiebige Druckstufe erforderlich, da eine Prothese ohne diese zum Stolpern führen könnte und/oder den Tragekomfort stark einschränkt.

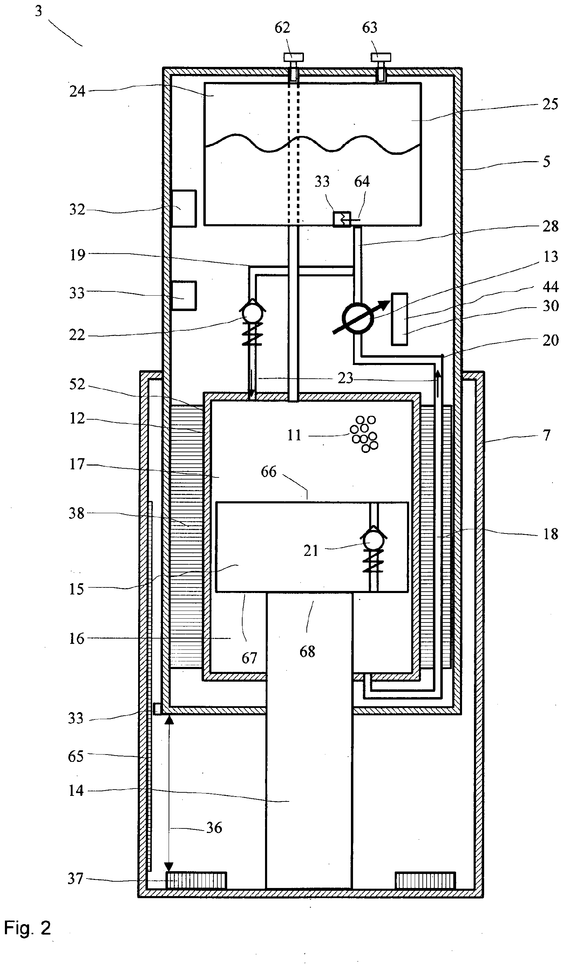

Fig. 2 zeigt eine schematische Querschnittsdarstellung des Rohrsystems 3 des Dämpfers aus Fig. 1.

Eine erste Komponente 5 als ein erstes Rohr (Standrohr) ist mit einem Ende des Dämpfers 1 bzw. der Dämpfereinrichtung 10 verbunden. Die zweite Komponente 7 als zweites Rohr ist mit dem anderen Ende der Dämpfereinrichtung bzw. mit der Kolbenstange verbunden. Die beiden Rohre 5 und 7 sind teleskopierbar vorgesehen und können hier aufeinander gleiten. Möglich ist aber auch eine Drehbewegung zweier Komponenten 5 und 7 zueinander. Der Dämpfer 1 kann neben der eigentlichen Dämpfereinrichtung 10 noch die erste Komponente 5 und die zweite Komponente 7 umfassen.

Die Dämpfereinrichtung 10 weist eine Dämpferkammer 12 auf, die durch einen Kolben 15 in eine erste Kammer 16 und eine zweite Kammer 17 aufgeteilt ist.

Der Kolben 15 ist mit einer Kolbenstange 14 versehen, die sich durch die erste Kammer 16 hindurch und aus dem Rohr 5 hinaus erstreckt. Das andere Ende der Kolbenstange ist mit dem unteren Ende des Rohres 7 als Tauchrohr 7 verbunden. Oberhalb der Dämpferkammer 12 ist das Drosselventil 13 angeordnet, welches elektrisch einstellbar ist. Dem Drosselventil 13 ist eine Felderzeugungseinrichtung 30 zugeordnet, die zur Erzeugung eines Magnetfeldes dient. Als Dämpfungsmedium 11 wird ein magnetorheologisches Fluid verwendet.

Ein erstes Einwegventil 21 ist in dem Kolben 15 vorgesehen, der ansonsten als Pumpkolben ausgeführt ist. Das Einwegventil 21 kann beispielsweise als Shim-Ventil ausgeführt sein und erlaubt nur die Strömung des magnetorheologischen Dämpfungsmediums 11 von der zweiten Kammer 17 durch den Kolben 15 in die erste Kammer 16 hinein, wenn der Druck innerhalb der zweiten Kammer 17 größer ist als innerhalb der ersten Kammer 16. In Gegenrichtung blockiert das Einwegventil 21.

Am hier unteren Ende der ersten Kammer 16 beginnt der Rückströmkanal 18, durch den das Dämpfungsmedium 11 von der ersten Kammer 16 zu dem Drosselventil 13 strömen kann. Das in Umlaufrichtung 23 strömende Dämpfungsmedium 11 durchströmt das Drosselventil 13, wo es entsprechend der Einstellungen der Magnetfeldquelle 30 bzw. Magneteinrichtung 30 gedämpft wird.

Der sich an das Drosselventil 13 anschließende Verbindungskanal 19 führt zu dem zweiten Einwegventil 22, welches in Umlaufrichtung 23 öffnet, wenn der Druck in dem Verbindungskanal 19 größer ist als der Druck in der zweiten Kammer 17. Von dem Verbindungskanal 19 zweigt hier der Ausgleichskanal 28 zu der Ausgleichskammer 24 ab, in der ein Ausgleichsvolumen 25 vorhanden ist. Das Ausgleichsvolumen 25 kann beispielsweise ein unter Überdruck stehender flexibler Balg oder ein Ballon oder dergleichen sein, der von dem Volumen der Ausgleichskammer 24 elastisch abgetrennt ist. Möglich ist auch der Einsatz eines Trennkolbens oder einer Feder zur Vorspannung.

Der Rückströmkanal 18 verläuft durch den Zwischenraum zwischen der Dämpferkammer 12 und der inneren Umfangsfläche des ersten Rohres als erster Komponente 5. Dort befindet sich in dem Zwischenraum eine Einsatzeinrichtung 38, die einen definierten Querschnitt für den Rückströmkanal 18 zur Verfügung stellt. Dadurch kann das Volumen des Dämpfungsmediums 11 erheblich reduziert werden, da nur noch der Querschnitt des Rückströmkanals 18 an der Einsatzeinrichtung 38 mit dem Dämpfungsmedium 11 gefüllt ist und nicht mehr der vollständige Zwischenraum. Dadurch kann das Gewicht der Dämpfereinrichtung 10 und des gesamten Dämpfers 1 erheblich gesenkt werden.

Eine Steuereinrichtung 32 dient zur Steuerung. Die Steuereinrichtung ist ein Computer, der die Steuerung, Regelung und Überwachung von Funktionen übernimmt. Dies kann eine offene Steuerung oder eine geschlossene Regelung sein, wobei der mit einem Sensor gemessene IST-Zustand mit einem berechneten SOLL-Zustand verglichen werden kann (Rückkopplung) und über den Dämpfer dann die Abweichung im geschlossenen Regelkreis minimiert wird. Die Steuereinrichtung kann einen 32-Bit-Mikrocontrollern ausgestattet sein, um die in Echtzeit benötigten Rechenoperationen genügend schnell und genau zu verarbeiten. Vorteilhaft kann aber auch sein, die Steuereinrichtung mit Field Programmable Gate Array zu ergänzen, da diese digitale Funktionen schneller ausführen. Die Steuereinrichtung hat integrierte Schnittstelle für analoge und digitale Eingangssignale von Sensoren und Ausgangssignale für die Dämpfer sowie der Feldbus (z.B. CAN-Bus) für die Kommunikation mit anderen Steuergeräten.

Die Steuerung 32 ist mit Sensoren 33 verbunden, die den aktuellen Zustand des Dämpfers erkennen und entsprechend darauf reagieren. Beispielsweise kann eine Sensoreinrichtung 33 den Federweg 36 in kurzen zeitlichen Abständen erfassen, sodass aus dem zeitlichen Ablauf der Signale auch auf Relativgeschwindigkeiten und somit auf Beschleunigungswerte geschlossen werden kann. Auch der Einsatz von Beschleunigungssensoren ist möglich. Der Federweg 36 kann über eine Positionserkennung der Sensoreinrichtung 33 relativ zu einer Maßeinrichtung 65 erfasst werden.

Endlagendämpfungen 37 können vorgesehen sein, um einen Anschlag des Dämpfers zu verhindern.

Im Betrieb führt ein Stoß zu einem Einfedern des Kolbens 15. Da das Dämpfungsmedium nach oben über das Einwegventil 22 nicht entweichen kann und da der Druck in der zweiten Kammer 17 steigt, öffnet das erste Einwegventil 21 und es strömt Dämpfungsmedium 11 von der zweiten Kammer durch das erste Einwegventil 21 in die erste Kammer 16.

Da beim Einfedern mehr Dämpfungsmedium in der zweiten Kammer 11 verdrängt wird, als in der ersten Kammer 16 zur Verfügung steht, muss das der eintauchenden Kolbenstange 14 entsprechende Volumen durch den Rückströmkanal 18 in Umlaufrichtung 23 zu dem Drosselventil 13 strömen, wo der Dämpfungskanal 31 des Drosselventils 13 dem Magnetfeld der Felderzeugungseinrichtung 30 ausgesetzt wird. Dadurch wird das Dämpfungsmedium entsprechend gedämpft.

Von dem Drosselventil 13 aus strömt das Dämpfungsmedium 11 ein kurzes Stück durch den Verbindungskanal 19 und dann durch den Ausgleichskanal 28 in die Ausgleichskammer 24 hinein. Das Einströmen des Dämpfungsmediums 11 kann durch den Detektor 64 der Sensoreinrichtung 33 am Einlass in die oder innerhalb der Ausgleichskammer 24 erfasst werden. Da sich das als Detektor 64 eingesetzte Detektorplättchen hier in Strömungsrichtung verbiegt oder verdreht, kann auf den Druckstufenfall zurückgeschlossen werden.

Beim Ausfedern, also im Zugstufenfall, bewegt sich der Kolben 15 in der Darstellung gemäß Fig. 2 nach unten und ein entsprechendes Stück der Kolbenstange 14 tritt aus der Dämpferkammer 12 wieder aus. Das sich in der ersten Kammer 16 befindende Dämpfungsmedium 11 kann nicht durch das nun blockierende Einwegventil 21 in die zweite Kammer 17 übertreten, sondern muss durch den Rückströmkanal 18 in die gleiche Umlaufrichtung 23 strömen, wie es in der Druckstufe der Fall war.

Das durch den Rückströmkanal 18 strömende Dämpfungsmedium 11 strömt durch das Drosselventil 13, wo es nun einem entsprechend angepassten Magnetfeld der elektrischen Spule 44 der Magnetfeldquelle 30 ausgesetzt wird.

Da im Zugstufenfall durch das Kolbenstangenvolumen bedingt nur weniger Dämpfungsmedium 11 aus der ersten Kammer 16 austritt, wie als Ausgleich in der zweiten Kammer 17 benötigt wird, öffnet das zweite Einwegventil 22 und es tritt aus der Ausgleichskammer 24 Dämpfungsmedium aus. Das Dämpfungsmedium tritt durch den Ausgleichskanal 28 und den Verbindungskanal 19 in die zweite Kammer 17 ein. Bei dem Austreten des Dämpfungsmediums 11 aus der Ausgleichskammer 24 wird das Detektorplättchen als Detektor der Sensoreinrichtung 33 entsprechend verformt, sodass auf dem Zugstufenfall zurückgeschlossen werden kann. Ein wichtiger Punkt dabei ist, dass aber im Zugstufenfall eine größere Menge des magnetorheologischen Dämpfungsmediums 11 durch das Drosselventil 13 durchtritt als bei gleichem Hub im Druckstufenfall. Das liegt daran, dass ein Teil des Dämpfungsmediums 11 in die Ausgleichskammer eingeleitet bzw. daraus entnommen wird.

Dadurch kann das Verhältnis der Steilheiten der Kennlinien in der Zugstufe und in der Druckstufe angepasst werden. Besonders vorteilhaft ist für viele Anwendungen, dass die Zugstufenkennlinie steiler eingestellt werden kann als die Druckstufenkennlinie. Die Einstellung kann konstruktiv über die Flächenverhältnisse eingestellt werden. Die Kolbenfläche 66 wirkt in der Druckstufe. In der Zugstufe wirkt die Kolbenfläche 67. Der Unterschied ergibt sich aus der Kolbenstangenfläche 68.

Bei der Bestimmung der Kennlinien ist aber zu beachten, dass innerhalb des Kreislaufes 20 in der Druckstufe nur ein Volumen proportional zu der der Kolbenstangenfläche 68 fließt. Der andere Anteil strömt nur durch das erste Einwegventil 21. Im Zugstufenfall strömt hingegen der Ringanteil des Kolbens, also die Kolbenfläche 67, die sich aus der Fläche 66 minus der Fläche 68 berechnet.

Je nach Durchmesser der Kolbenstange 68 und des Durchmessers des Kolbens 15 können die Strömungsverhältnisse und damit die Kennliniensteigungen im Druck- und Zugstufenfall variiert werden.

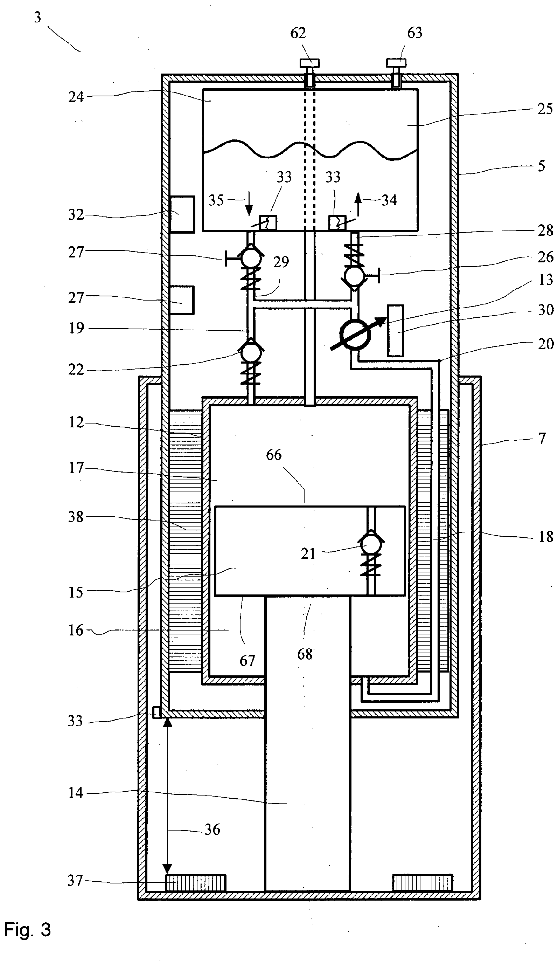

Fig. 3 zeigt ein weiteres Ausführungsbeispiel, wobei in einem ebenfalls stark schematischen Querschnitt das Rohrsystem 3 eines Dämpfers 1 gemäß Fig. 1 dargestellt ist. Grundsätzlich ist das Rohrsystem 3 gemäß Fig. 3 ähnlich aufgebaut wie das Rohrsystem 3 gemäß Fig. 2. Im Unterschied zu der Darstellung gemäß Fig. 2 wird der Verbindungskanal 19 nach dem Drosselventil 13 aber in zwei Ausgleichskanäle 28 und 29 zum Austausch mit der Ausgleichskammer 24 aufgeteilt.

In dem ersten Ausgleichskanal 28 von dem Drosselventil 13 zur Ausgleichskammer 24 hin ist ein erstes Rückschlagventil 26 vorgesehen, welches nur einen Durchfluss des Dämpfungsmediums 11 von dem Drosselventil 13 in die Ausgleichskammer 28 hinein erlaubt. Eine Sensoreinrichtung 33 am Eingang der Ausgleichskammer kann die Richtung 34 der Relativbewegung erkennen und damit auf den Druckstufenfall schließen.

Damit das Dämpfungsmedium 11 aus der Ausgleichskammer 24 austreten kann, ist der zweite Ausgleichskanal 29 vorgesehen, an dem ein zweites Rückschlagventil 27 angeordnet ist. Dieses Rückschlagventil 29 öffnet nur, wenn der Druck in der Ausgleichskammer 24 größer ist als der Druck in dem Verbindungskanal 19.

Hier im Ausführungsbeispiel sind die Rückschlagventile 26 und 27 einstellbar. Es ist möglich, dass Bedienelemente außen am Dämpfer vorgesehen sind, sodass die Rückschlagventile 26 und 27 gegebenenfalls vom z. B. Prothesenträger bedienbar sind. Dazu können entsprechend Einstellräder vorgesehen sein. Möglich ist auch eine elektrische Fernsteuerung.

Bei diesen Ausgestaltungen ist es so, dass das Drosselventil 13 ein elektrisch einstellbares Drosselventil ist, und dass als Dämpfungsmedium 11 ein magnetorheologisches Fluid verwendet wird.

Obwohl der Dämpfer 1 gemäß Fig. 3 auch mit einem magnetorheologischen Fluid betrieben wird, kann es dennoch günstig sein, die Rückschlagventile 25 und 26 einstellbar oder (vor-) justierbar vorzusehen, da damit eine Anpassung an eine Grundkurve erfolgen kann. Situationsabhängig kann dann eine Einstellung des Drosselventils 13 erfolgen.

Wie schon in Fig. 1 dargestellt, sind auch in Fig. 2 und Fig. 3 Ventile 62 und 63 vorgesehen. Das Ventil 62 kann zum Nachfüllen oder zum Austauschen von Dämpfungsmedium 11 dienen, während das Ventil 63 beispielsweise zur Überprüfung des Luftdrucks in dem Ausgleichsvolumen 25 der Ausgleichskammer 24 oder aber zum Nachfüllen von Druckluft dient.

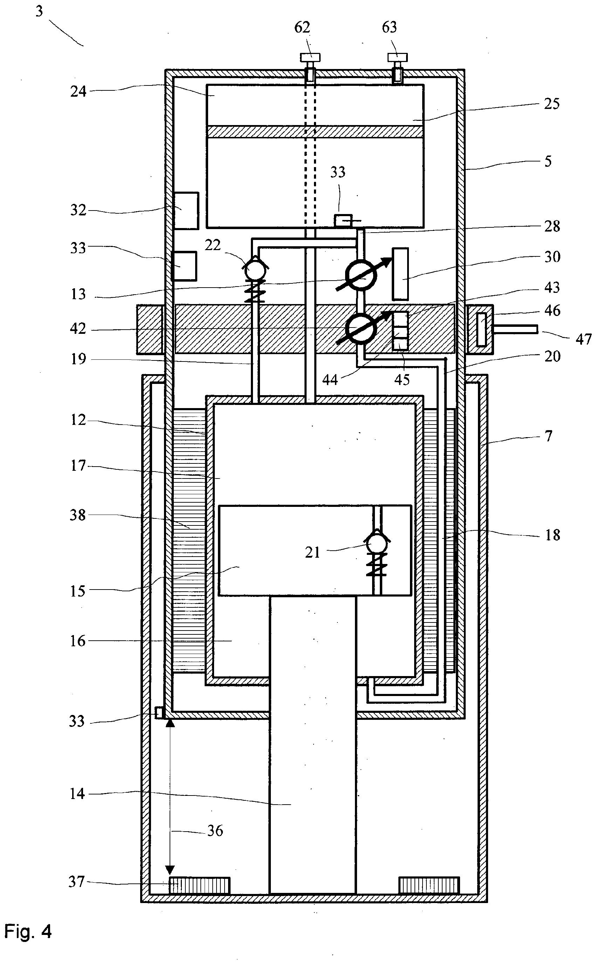

Fig. 4 zeigt ein anderes Rohrsystem 4 für einen Dämpfer 1 gemäß Fig. 1. Auch dieses Rohrsystem 4 ist grundsätzlich ähnlich aufgebaut wie das Rohrsystem 4 nach den Fig. 2 und 3.

Im Unterschied zur Ausgestaltung gemäß Fig. 2 weist das Rohrsystem gemäß Fig. 4 noch ein weiteres Drosselventil oder Absenkventil 42 auf, welches in Reihe und hier vor dem Drosselventil 13 angeordnet ist. Hier ist die Dämpfereinrichtung 10 mit einem magnetorheologischen Fluid als Dämpfungsmedium 11 ausgerüstet, sodass die Magnetfeldquellen 30 und 43 für das Drosselventil 13 und das Absenkventil 42 vorgesehen sind.

Die Magnetfeldquelle 43 bzw. Magneteinrichtung 43 nach Fig. 4 kann ebenfalls eine elektrische Spule 44 umfassen, die ein entsprechendes Magnetfeld erzeugt. Es ist auch möglich, dass beispielsweise ein Remanenzmagnet 45 vorgesehen ist, dessen Feldstärke bei Bedarf oder in periodischen Abständen durch magnetische Impulse der elektrischen Spule 44 auf den aktuell gewünschten Wert eingestellt wird. Damit kann ein dauerhaftes Magnetfeld in dem Remanenzmagneten 45 erzeugt werden, welches auch nach Abschalten des für die elektrische Spule 44 benötigten Stroms zur Verfügung steht. Bei Bedarf kann die magnetische Feldstärke der Magnetfeldquelle 43 noch durch ein magnetisches Feld der elektrischen Spule 44 modifiziert werden.

Alternativ oder zusätzlich dazu kann noch ein Permanentmagnet 46 an einem äußeren Bedienhebel vorgesehen sein, der zum Beispiel um das Standrohr 5 herum drehbar angeordnet ist. Durch Positionierung des Permanentmagneten 46 derart, dass dessen Magnetfeld das Absenkventil 42 in gewünschter Weise mit einem Magnetfeld beaufschlagt, kann in dem Absenkventil 42 ein entsprechendes Magnetfeld erzeugt werden. Durch Wegdrehen wirkt das Magnetfeld nicht mehr auf das Absenkventil 42 ein.

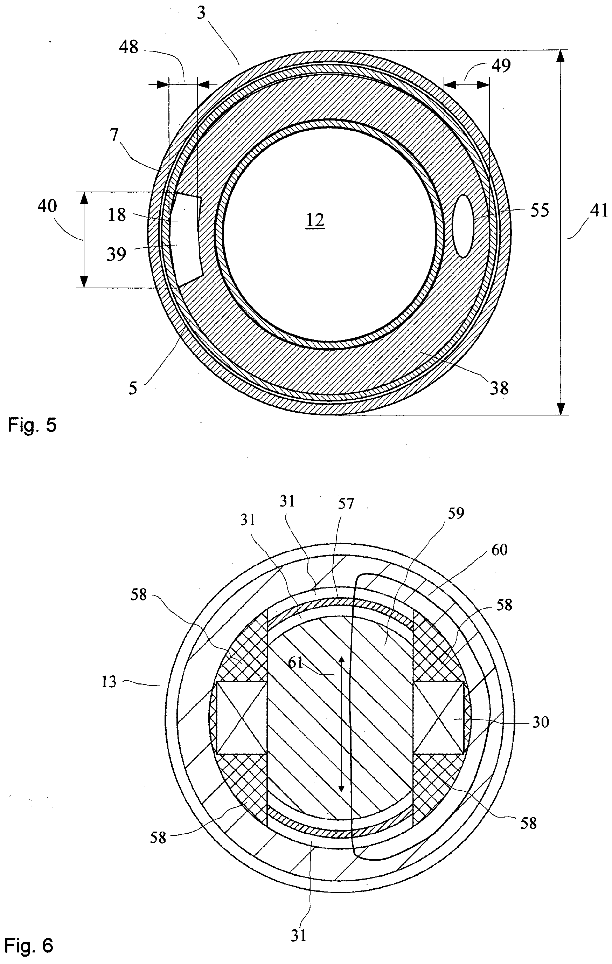

Im einfachsten Fall wird nur eine elektrische Spule 44 zur Felderzeugung eingesetzt. Dann ist es auf einfache Art und Weise möglich, eine einmal ein gewisses Stück eingefederter Dämpfer am selbsttätigen Ausfedern zu hindern. Das erfolgt dadurch, dass im Zugstufenfall immer ein zusätzliches Magnetfeld an dem Absenkventil 42 erzeugt wird, was das Ausfedern zusätzlich dämpft. Das führt im Resultat zu einem dauerhaft abgesenkten Dämpfer, der beispielsweise beim Sitzen (Prothese) von Vorteil ist. Fig. 5 zeigt einen typischen Querschnitt durch ein Rohrsystem nach den Fig. 2, 3 oder 4. Dabei ist in der Mitte die ungeschnittene Dämpferkammer 12 zu erkennen. Radial außen ist das Standrohr 5 des Rohrsystems 3 im Schnitt dargestellt. Radial nach außen schließt sich das gegenüber dem Standrohr 5 teleskopierbare Tauchrohr 7 an.

Zu der Dämpfereinrichtung 10 bzw. der Dämpferkammer 12 ergibt sich ein radialer Abstand 49, der hier praktisch vollständig durch eine Einsatzeinrichtung 38 gefüllt ist. Die Einsatzeinrichtung 38 kann einteilig sein, kann aber auch aus zwei oder mehr Teilen gebildet werden. Die Einsatzeinrichtung 38 erstreckt sich in den Ausführungsbeispielen im Wesentlichen über die Länge der Dämpferkammer 12, kann aber auch länger oder kürzer sein.

Auf der einen Seite ist ein Rückströmkanal 18 an der Einsatzeinrichtung 38 vorgesehen. Der Rückströmkanal 18 dient hier als Rückströmkanal für das Dämpfungsmedium 11 auf dem Weg von der ersten Kammer 11 über das Drosselventil in die Ausgleichskammer 24 oder in die zweite Kammer 17. Der Rückströmkanal 18 kann etwa die hier dargestellte Form oder aber andere Formen wie runde, quadratische oder rechteckige Formen aufweisen. Grundsätzlich ist auch jede andere Form möglich wie beispielsweise eine elliptische Form.

Besonders vorteilhaft ist es, wenn das Verhältnis der Querschnittsfläche als Strömungsquerschnitt 39 zu dem Umfang des Rückströmkanals 18 groß ist, sodass der Strömungswiderstand des Dämpfungsmediums 11 in dem Rückströmkanal 18 auch bei hohen Strömungsgeschwindigkeiten relativ gering bleibt. Dazu ist das Verhältnis des größten Durchmessers bzw. der längsten Erstreckung 40 zu der Breite 48 hier relativ klein. Insbesondere ist die Länge 40 kleiner als der Durchmesser 41 des Rohrsystems und insbesondere kleiner als der Radius des Rohrsystems, vorzugweise auch kleiner als der halbe Radius des Rohrsystems. Andererseits ist der Strömungsquerschnitt 39 so groß wie nötig.

Auf der anderen Seite kann ein gleichartiger gestalteter Kanal 55 vorgesehen sein. Möglich ist, dass beide Kanäle z. B. einen rechteckigen oder elliptischen Querschnitt aufweisen. Der andere Kanal 55 kann beispielsweise zur Durchführung elektrischer Leitungen oder dergleichen dienen. Möglich ist es auch, dass beide Kanäle als Rückstromkanäle eingesetzt werden.

Insgesamt kann die Einsatzeinrichtung massiv ausgeführt sein und es ist auch möglich, dass die Einsatzeinrichtung 38 hohle Bereiche oder Hohlkammern aufweist, sodass die durchschnittliche Dichte der Einsatzeinrichtung 38 reduziert wird. Die Einsatzeinrichtung kann wenigstens teilweise aus Metall und/oder Kunststoff bestehen.

Die durchschnittliche Dichte der Einsatzeinrichtung 38 wenigstens im Bereich des Zwischenraums zwischen dem Rohrsystem 4 und der Dämpferkammer 12 ist geringer als die Dichte des Dämpfungsmediums 11 und insbesondere höchstens halb so groß. Dadurch kann ein erheblicher Gewichtsanteil eingespart werden. Versuche haben gezeigt, dass das Gewicht der Dämpfereinrichtung um erheblich mehr als 10 % gesenkt werden konnte. Auch 20 % und mehr können möglich sein.

Fig. 6 zeigt einen schematischen Querschnitt eines beispielhaft dargestellten Drosselventils 13. Innerhalb des hier dargestellten Drosselventils 13 ist zentral ein Kern 59 vorgesehen, der von einer gewickelten elektrischen Spule als Felderzeugungseinrichtung 30 umgeben ist. Hier sind insgesamt vier Dämpfungskanäle 31 vorgesehen, von denen jeweils zwei durch einen Fächer oder eine fächerartige Struktur 57 voneinander getrennt sind. Dadurch wird die Wirksamkeit erhöht.

Die Feldlinien 61 bei angelegtem magnetischen Feld verlaufen durch den Kern 59, treten etwa senkrecht durch einen Dämpfungskanal 31, den sich anschließenden Fächer 57 und den zweiten Dämpfungskanal 31 hindurch und werden durch den Ring 60 aus einem magnetisch leitfähigen Material, hier etwa im Halbkreis um den Kern herum gelenkt bis im unteren Bereich wieder zwei Dämpfungskanäle 31 mit dazwischen liegender Fächerwand 57 vorgesehen sind, durch den die Feldlinien etwa senkrecht durchtreten, sodass insgesamt geschlossene Feldlinien 61 vorliegen. In Fig. 4 ist nur eine Feldlinie stellvertretend dargestellt.

Benachbart zu einer elektrischen Spule 30 sind magnetische Isoliermaterialien 58 vorgesehen, um das magnetische Feld, wie gewünscht zu formen.

Fig. 7 zeigt einen schematischen Querschnitt durch ein Rohrsystem mit einem Drosselventil. Das Rohrsystem kann in einem der zuvor beschriebenen anderen Ausgestaltungen verwendet werden. In dem Standrohr 5 ist ein Zylinder mit einer Wandung 52 angeordnet. Die Wandung 52 begrenzt die Dämpferkammer 12. Zwischen dem Zylinder und dem Standrohr 5 ist eine Spule als Magnetfeldquelle 30 vorgesehen. Die Spule ist um den Ringkern 59 gewickelt. Der Ringkern 59 weist an eine Stelle einen Schlitz auf. Dieser Schlitz bildet den Dämpfungsspalt 55, durch den das magnetorheologische Fluid durchtreten muss. Der Dämpfungsspalt 55 wird mit dem durch die elektronische Steuereinrichtung gesteuerten Magnetfeld beaufschlagt. Beispielhaft sind einige Magnetfeldlinien 61 eingezeichnet, die den Dämpfungsspalt 55 etwa senkrecht durchsetzen. Diese Ausgestaltung ermöglicht einen effizienten Dämpfer. Im Inneren kann die Dämpferkammer 12 vorgesehen sein.

Diese Ausgestaltung nach Fig. 7 mit der hier dargestellten langen dünnen Zylinderspule bietet erhebliche Vorteile. Die magnetische Spannung lässt sich über das Produkt aus Windungszahl und Spulenstrom berechnen. Die Spule soll nun in dem Magnetkreis ein definiertes Magnetfeld erzeugen, z. B. einen bestimmten magnetischen Fluss oder ein Feld bestimmter magnetischer Feldstärke. Gemäß der Formel kann die Spule auch als eine einzelne Windung berechnet werden, in der ein entsprechend hoher Strom fließt (nämlich der Spulenstrom multipliziert mit der Windungszahl).

Für einen Magnetkreis ist es im Wesentlichen unerheblich, ob wenig Strom auf zahlreichen dünnen Windungen oder entsprechend mehr Strom auf wenigen oder gar einer einzelnen Windung um den Kern kreist, solange der Spulenstrom multipliziert mit der Windungszahl konstant bleibt.

Die Verluste ergeben sich aus dem Strom zum Quadrat und dem elektrischen Widerstand. Die einzelnen Leiter lassen sich auch nicht beliebig dicht aneinander packen und bedingt durch die Isolation und den geometrischen Aufbau hat die Spule einen (Kupfer-) Füllfaktor unter 100%. Ausgehend von einem vorgegebenen Bauraum und gleicher Windungszahl hat folglich eine Spule mit höherem Füllfaktor mehr leitfähiges Material (=dickere Drähte), wodurch der Widerstand und somit die Verlustleistung sinken. Ausgehend von gleicher Windungszahl und gleicher Drahtstärke bedeutet ein höherer Füllfaktor eine kleinere Spule. Dadurch sinkt in der Regel die mittlere Windungslänge und somit auch der elektrische Widerstand. Deshalb ist mit der in Fig. 7 dargestellten Konstruktion ein besonders geringer Energieverbrauch erzielbar, der insbesondere bei Outdoorprodukten sehr wichtig und vorteilhaft ist.

In allen Ausführungsformen ist die Magnetfeldquelle bzw. Magneteinrichtung 30 vorzugsweise insgesamt außerhalb der ersten und zweiten Kammer 16, 17 und insbesondere auch außerhalb der ganzen Dämpferkammer 12, des Kolbens 15 und der Kolbenstange 14 angeordnet. Die Magneteinrichtung 30 wird in allen Fällen immer von derselben Seite aus angeströmt. Das magnetorheologische Dämpfungsmedium 11 strömt im Koben/Zylinderraum nur in eine Richtung innerhalb des Einwegkreislaufes 20.

Vorteilhaft ist auch, dass das magnetorheologische Dämpfungsmedium 11 aufgrund des Einwegkreislaufs 20 immer durchmischt wird.

Die außerhalb der ersten und zweiten Kammer 16, 17 angebrachte elektrische Spule 44 ist derart angeordnet, dass das erzeugte Magnetfeld wenigstens teilweise durch den Dämpfungskanal des Drosselventils verläuft. Insbesondere ist die elektrische Spule 44 derart angeordnet, dass eine Symmetrieachse der elektrischen Spulen quer zu der Strömungsrichtung des magnetorheologischen Dämpfungsmediums ausgerichtet ist.

Ein Dämpfer wie zuvor beschrieben ist insbesondere, für einen Sitz eines Fahrzeugs wie insbesondere eines PKW, LKW, Bus oder eines anderen Nutzfahrzeuges oder eines Militärfahrzeugs, Truppenfahrzeugs, eines Panzers, Helikopters, eines Landfahrzeugs oder einer Baumaschine vorgesehen und geeignet.

Insgesamt stellt die Erfindung einen vorteilhaften Dämpfer zur Verfügung, der durch den Einsatz von magnetorheologischen Fluiden sehr vorteilhafte Eigenschaften aufweist. Es wird ein großer Hub ermöglicht, da die Einbauten in dem Rohrsystem 3 kleinbauend ausgestaltet werden können. Es wird im Normalfall nur ein einziges Drosselventil 13 benötigt, um sowohl im Druckstufenfall als auch im Zugstufenfall effektiv und unterschiedlich dämpfen zu können.

Durch die Verwendung einer Einsatzeinrichtung kann das Gesamtgewicht der einsatzfähigen Dämpfer um fast 5 % oder mehr gesenkt werden, was die Attraktivität für den z. B. Prothesenträger deutlich erhöht.

**Bezugszeichenliste:**

| | | | |
|---|---|---|---|
| 1 | Dämpfer | 38 | Einsatzeinrichtung |
| 3 | Rohrsystem | 39 | Strömungsquerschnitt |
| 5 | Standrohr | 40 | Durchmesser, Länge |
| 7 | Tauchrohr | 41 | Durchmesser |
| 10 | Dämpfereinrichtung | 42 | Absenkventil |
| 11 | Dämpfungsmedium | 43 | Magnetfeldquelle, Magneteinrichtung |
| 12 | Dämpferkammer | | |
| 13 | Drosselventil | 44 | elektrische Spule |
| 14 | Kolbenstange | 45 | Remanenzmagnet |
| 15 | Kolben, Pumpkolben | 46 | Permanentmagnet |
| 16 | erste Kammer | 47 | Absenkstellung |
| 17 | zweite Kammer | 48 | Breite |
| 18 | Rückströmkanal | 49 | Abstand |
| 19 | Verbindungskanal | 50 | Federeinrichtung |
| 20 | Einwegkreislauf | 55 | Kanal |
| 21 | erstes Einwegventil | 57 | Fächer |
| 22 | zweites Einwegventil | 58 | Isoliermaterial |
| 23 | Umlaufrichtung | 59 | Kern |
| 24 | Ausgleichskammer | 60 | Ring |
| 25 | Ausgleichsvolumen | 61 | Feldlinie |
| 26 | erstes Rückschlagventil | 62 | Ventil? |
| 27 | zweites Rückschlagventil | 63 | Ventil? |
| | | 64 | Detektor |
| 28 | Ausgleichskanal | 65 | Maßeinrichtung |
| 29 | zweiter Ausgleichskanal | 66 | Kolbenfläche Druckstufe |
| 30 | Magnetfeldquelle, Magneteinrichtung | 67 | Kolbenfläche Zugstufe |
| | | 68 | Kolbenstangenfläche |
| 31 | Dämpfungskanal | 76 | Beinprothese |
| 32 | Steuereinrichtung | | |
| 33 | Sensoreinrichtung | | |
| 34 | Richtung | | |
| 35 | Richtung | | |
| 36 | Federweg | | |
| 37 | Endlagendämpfung | | |

## Patentansprüche

1. Dämpfer (1) mit wenigstens einer Dämpferkammer (12) und einem steuerbares Drosselventil (13), wobei die Dämpferkammer (12) durch einen beweglichen und mit einer Kolbenstange (14) verbundenen Kolben (15) in eine erste Kammer (16) und in eine zweite Kammer (17) geteilt ist, und wobei die erste Kammer (16) über einen Rückströmkanal (18) und das Drosselventil (13) mit der zweiten Kammer (17) verbunden ist,
wobei eine elektronische Steuereinrichtung (32) vorgesehen ist und wobei dem Drosselventil (13) eine durch die elektronische Steuereinrichtung (32) gesteuerte Magnetfeldquelle (30) zugeordnet ist, um ein durch wenigstens einen Dämpfungskanal (31) des Drosselventils (13) strömendes magnetorheologisches Dämpfungsmedium (11) wenigstens teilweise mit einem magnetischem Feld zu beaufschlagen, und wobei eine Ausgleichskammer (24) mit einem vorgespannten Ausgleichsvolumen (25) vorgesehen ist, wobei das Ausgleichsvolumen (25) mit dem Drosselventil (13) und der zweiten Kammer (17) verbunden ist, und wobei das Drosselventil (13), die Magnetfeldquelle und die Ausgleichskammer (24) extern der Dämpferkammer (12) angeordnet sind, wobei für das magnetorheologische Dämpfungsmedium (11) im Wesentlichen ein Einwegkreislauf (20) vorgesehen ist, in welchem wenigstens zwei Einwegventile (21, 22) vorgesehen sind, sodass sowohl beim Eintauchen der Kolbenstange (14) in die Dämpferkammer (12) hinein als auch beim Austauchen der Kolbenstange (14) aus der Dämpferkammer (12) heraus das Dämpfungsmedium (11) in der gleichen Umlaufrichtung (23) umströmt, wobei ein erstes der Einwegventile (21) an dem Kolben (15) angeordnet ist, welches eine Strömung des Dämpfungsmediums (11) von der zweiten Kammer (17) in die erste Kammer (16) erlaubt, und wobei zwischen dem Drosselventil (13) und der zweiten Kammer (17) ein zweites Einwegventil (22) angeordnet ist, welches eine Strömung des Dämpfungsmediums (11) von dem Drosselventil (13) in die zweite Kammer (17) erlaubt, **dadurch gekennzeichnet, dass** ein weiteres Drosselventil mit einer weiteren Magnetfeldquelle (43) vorgesehen ist und wobei das weitere Drosselventil in Reihe zu dem Drosselventil (13) geschaltet ist.

2. Dämpfer (1) nach Anspruch 1, wobei das Drosselventil (13) axial benachbart zu der Dämpferkammer (12) angeordnet ist und wobei die Ausgleichskammer (24) axial beabstandet von dem Kolben angeordnet ist und insbesondere axial benachbart zu der Dämpferkammer (12) und vorzugsweise axial neben der zweiten Kammer (17) angeordnet ist.

3. Dämpfer (1) nach dem vorhergehenden Anspruch, wobei das Drosselventil (13) über ein erstes Rückschlagventil (26) mit der Ausgleichskammer (24) verbunden ist, welches nur eine Strömung des Dämpfungsmediums (11) von dem Drosselventil (13) in die Ausgleichskammer (24) erlaubt und/oder wobei die Ausgleichskammer (24) über ein zweites Rückschlagventil (27) mit der zweiten Kammer (17) verbunden ist, welches nur eine Strömung des Dämpfungsmediums (11) von der Ausgleichskammer (24) in die zweite Kammer (17) erlaubt.

4. Dämpfer (1) nach einem der vorhergehenden Ansprüche, wobei ein Verhältnis des Außendurchmessers der Kolbenstange zu dem Außendurchmesser des Kolbens (14) zwischen 0,2 und 0,4 beträgt, und/oder wobei ein Verhältnis des Außendurchmessers der Kolbenstange zu dem Außendurchmesser des Kolbens (14) an ein vorgegebenes Verhältnis einer Grunddämpfung in der Druckstufe und einer Grunddämpfung in der Zugstufe angepasst ist.

5. Dämpfer (1) nach einem der vorhergehenden Ansprüche, wobei die Magnetfeldquelle eine außerhalb der ersten und zweiten Kammer angebrachte liegende elektrische Spule umfasst, bei der eine Spulenachse quer zu der Strömungsrichtung des magnetorheologischen Dämpfungsmediums ausgerichtet ist.

6. Dämpfer (1) nach dem vorhergehenden Anspruch, wobei wenigstens eine Sensoreinrichtung (33) vorgesehen ist, welche insbesondere zur Erkennung wenigstens einer Stellgröße vorgesehen ist.

7. Dämpfer (1) nach dem vorhergehenden Anspruch, wobei die Sensoreinrichtung (33) dazu vorgesehen ist, ein Maß für eine Relativgeschwindigkeit zu erfassen und insbesondere für eine Relativgeschwindigkeit des Kolbens (15) zu der Dämpferkammer (12) zu erfassen und/oder wobei eine Sensoreinrichtung (33) dazu vorgesehen ist, eine Richtung (34, 35) der Relativbewegung zwischen dem Kolben (15) und der Dämpferkammer (12) zu erfassen und/oder wobei eine Sensoreinrichtung (33) dazu vorgesehen ist, ein Maß für einen Federweg (36) und/oder wenigstens eine Beschleunigung zu erfassen.

8. Dämpfer (1) nach einem der vorhergehenden Ansprüche, wobei im bestimmungsgemäßen Gebrauch die erste Kammer (16) unterhalb der zweiten Kammer (17) und das Drosselventil (13) oberhalb der Dämpferkammer (12) angeordnet ist und wobei die Ausgleichskammer (24) oberhalb des Drosselventils (13) vorgesehen ist.

9. Dämpfer (1) nach einem der vorhergehenden Ansprüche, wobei wenigstens die Dämpferkammer und das Drosselventil in einem Rohrsystem angeordnet sind und wobei eine Einsatzeinrichtung (38) zwischen dem Rohrsystem (3) und der Dämpferkammer (12) vorgesehen ist, wobei der Rückströmkanal (18) wenigstens abschnittsweise an der Einsatzeinrichtung (38) vorgesehen ist und vorzugsweise den Strömungsquerschnitt (39) des Rückströmkanals (18) begrenzt, wobei eine mittlere Dichte der Einsatzeinrichtung (38) zwischen dem Rohrsystem (3, 4) kleiner als eine mittlere Dichte des Dämpfungsmediums (11) ist.

10. Dämpfer (1) nach dem vorhergehenden Anspruch, wobei ein größter Durchmesser (40) eines Strömungsquerschnittes (39) des Rückströmkanals (18) an der Einsatzeinrichtung (38) kleiner als ein Durchmesser (41) und insbesondere kleiner als ein Radius des Rohrsystems (3) ist.

11. Dämpfer (1) nach einem der vorhergehenden Ansprüche, wobei an dem Drosselventil (13) die Strömung des magnetorheologischen Dämpfungsmediums mittels der Magnetfeldquelle variiert und deren Schaltzustand dann stromlos gehalten werden kann.

12. Dämpfer (1) nach einem der vorhergehenden Ansprüche, wobei das weitere Drosselventil als Absenkventil (42) ausgebildet ist und wobei vorzugsweise die weitere Magnetfeldquelle (43) eine elektrische Spule (44) und/oder einen Remanenzmagneten (45) und/oder wenigstens einen Permanentmagneten (46) umfasst und wobei insbesondere wobei der Permanentmagnet (46) wenigstens zwischen einer Normalstellung und einer Absenkstellung (47) beweglich ist.

13. Dämpfer (1) nach einem der vorhergehenden Ansprüche, für eine Prothese.

14. Dämpfer (1) nach einem der vorhergehenden Ansprüche, für ein motorbetriebenes Zweirad.

15. Dämpfer (1) nach einem der vorhergehenden Ansprüche, für einen Sitz eines Fahrzeugs wie insbesondere eines PKW, LKW, Bus oder eines anderen Nutzfahrzeuges oder eines Militärfahrzeugs, Truppenfahrzeugs, eines Panzers, Helikopters, eines Landfahrzeugs oder einer Baumaschine.

## Claims

1. Damper (1) with at least one damper chamber (12) and a controllable throttle valve (13) wherein the damper chamber (12) is partitioned by a piston (15) that is movable and connected with a piston rod (14) into a first chamber (16) and a second chamber (17), and wherein the first chamber (16) is connected with the second chamber (17) via a return duct (18) and the throttle valve (13), wherein an electronic control device (32) is provided and wherein a magnetic field source (30) controlled by the electronic control device (32) is assigned to the throttle valve (13) to apply a magnetic field on at least part of a magnetorheological damping medium (11) that flows through at least one damping duct (31) of the throttle valve (13), and wherein an equalizing chamber (24) with a preloaded equalizing volume (25) is provided wherein the equalizing volume (25) is connected with the throttle valve (13) and the second chamber (17), and wherein the throttle valve (13), the magnetic field source and the equalizing chamber (24) are disposed external of the damper chamber (12), wherein a one-way circuit (20) is substantially provided for the magnetorheological damping medium (11) in which circuit at least two one-way valves (21, 22) are provided such that both when the piston rod (14) plunges into the damper chamber (12) and when the piston rod (14) emerges out of the damper chamber (12) the damping medium (11) flows in the same direction of circulation (23) wherein a first of the one-way valves (21) is disposed at the piston (15), allowing a flow of the damping medium (11) from the second chamber (17) into the first chamber (16), and wherein a second one-way valve (22) is disposed between the throttle valve (13) and the second chamber (17) allowing the damping medium (11) to flow from the throttle valve (13) into the second chamber (17), **characterized in that** another throttle valve with another magnetic field source (43) is provided and wherein the other throttle valve is connected in series with the throttle valve (13).

2. The damper (1) according to claim 1 wherein the throttle valve (13) is disposed axially adjacent to the damper chamber (12) and wherein the equalizing chamber (24) is disposed axially spaced apart from the piston and in particular axially adjacent to the damper chamber (12) and preferably axially adjacent to the second chamber (17).

3. The damper (1) according to the preceding claim wherein the throttle valve (13) is connected with the equalizing chamber (24) via a first check valve (26) which only allows flow of the damping medium (11) from the throttle valve (13) into the equalizing chamber (24) and/or wherein the equalizing chamber (24) is connected with the second chamber (17) via a second check valve (27) which only allows flow of the damping medium (11) from the equalizing chamber (24) into the second chamber (17) .

4. The damper (1) according to any of the preceding claims wherein a relationship of the outer diameter of the piston rod to the outer diameter of the piston (14) lies between 0.2 and 0.4, and/or wherein a relationship of the outer diameter of the piston rod to the outer diameter of the piston (14) is adapted to a specified relationship of a basic damping in the compression stage and a basic damping in the rebound stage.

5. The damper (1) according to any of the preceding claims wherein the magnetic field source comprises a horizontal electric coil disposed external of the first and second chambers where a coil axis is oriented transverse to the flow direction of the magnetorheological damping medium.

6. The damper (1) according to the preceding claim wherein at least one sensor device (33) is provided which is in particular provided for recognizing at least one controlled variable.

7. The damper (1) according to the preceding claim wherein the sensor device (33) is provided for capturing a rate for a relative speed and in particular for a relative speed of the piston (15) to the damper chamber (12) and/or wherein a sensor device (33) is provided for capturing a direction (34, 35) of the relative motion between the piston (15) and the damper chamber (12) and/or wherein a sensor device (33) is provided for capturing a measure for a suspension travel (36) and/or at least one acceleration.

8. The damper (1) according to any of the preceding claims wherein in use as intended the first chamber (16) is disposed beneath the second chamber (17) and the throttle valve (13), above the damper chamber (12) and wherein the equalizing chamber (24) is provided above the throttle valve (13).

9. The damper (1) according to any of the preceding claims wherein at least the damper chamber and the throttle valve are disposed in a tube system and wherein an insert device (38) is provided between the tube system (3) and the damper chamber (12), wherein at least portions of the return duct (18) are provided at the insert device (38) and preferably define the flow cross-section (39) of the return duct (18), wherein a mean density of the insert device (38) between the tube system (3, 4) is lower than a mean density of the damping medium (11).

10. The damper (1) according to the preceding claim wherein a largest diameter (40) of a flow cross-section (39) of the return duct (18) at the insert device (38) is smaller than a diameter (41) and in particular smaller than a radius of the tube system (3) .

11. The damper (1) according to any of the preceding claims wherein the flow of the magnetorheological damping medium is variable by means of the magnetic field source and its switch status can then be maintained currentless at the throttle valve (13).

12. The damper (1) according to any of the preceding claims wherein the further throttle valve is configured as a lowering valve (42) and wherein preferably the further magnetic field source (43) comprises an electric coil (44) and/or a remanence magnet (45) and/or at least one permanent magnet (46) and wherein in particular the permanent magnet (46) is movable at least between a normal position and a lowered position (47).

13. The damper (1) according to any of the preceding claims for a prosthesis.

14. The damper (1) according to any of the preceding claims for a motor-driven two-wheeled vehicle.

15. The damper (1) according to any of the preceding claims for a seat of a vehicle such as in particular a car, truck, bus or other utility vehicle or a military vehicle, troop vehicle, tank, helicopter, land vehicle or construction machine.

## Revendications

1. Amortisseur (1) doté d'au moins une chambre d'amortisseur (12) et d'une soupape d'étranglement (13) susceptible d'être commandée, ladite chambre d'amortisseur (12) étant divisée en une première chambre (16) et en une deuxième chambre (17) par un piston (15) mobile et relié à une tige de piston (14), et ladite première chambre (16) étant reliée à ladite deuxième chambre (17) via un canal de reflux (18) et ladite soupape d'étranglement (13), un organe de commande électronique (32) étant prévu et une source de champ magnétique (30), commandée par ledit organe de commande électronique (32), étant associée à ladite soupape d'étranglement (13) pour appliquer au moins partiellement un champ magnétique à un agent amortisseur (11) magnéto-rhéologique qui passe au moins par un canal d'amortissement (31) de ladite soupape d'étranglement (13), et une chambre d'équilibrage (24) étant prévue avec un volume d'équilibrage (25) dé précontrainte, ledit volume d'équilibrage (25) étant relié à ladite soupape d'étranglement (13) et à ladite deuxième chambre (17), et ladite soupape d'étranglement (13), ladite source de champ magnétique et ladite chambre d'équilibrage (24) étant agencées à l'extérieur de la chambre d'amortisseur (12), étant prévu sensiblement pour l'agent amortisseur (11) magnéto-rhéologique un circuit anti-retour (20) dans lequel sont prévus au moins deux vannes anti-retour (21, 22) de sorte qu'aussi bien lorsque ladite tige de piston (14) pénètre dans ladite chambre d'amortisseur (12) que lorsque ladite tige de piston (14) ressort de ladite chambre d'amortisseur (12), ledit agent amortisseur (11) circule dans le même sens (23), une première desdites vannes anti-retour (21) étant agencée sur ledit piston (15), permettant un passage dudit agent amortisseur (11) de ladite deuxième chambre (17) dans ladite première chambre (16) et une deuxième vanne anti-retour (22) étant agencée entre ladite soupape d'étranglement (13) et ladite deuxième chambre (17), permettant un passage dudit agent amortisseur (11) de ladite soupape d'étranglement (13) dans ladite deuxième chambre (17), **caractérisé en ce qu'**une autre soupape d'étranglement est prévue dotée d'une autre source de champ magnétique (43) et ladite autre soupape d'étranglement étant connectée en série à ladite soupape d'étranglement (13).

2. Amortisseur (1) selon la revendication 1, la soupape d'étranglement (13) étant agencée axialement adjacente à la chambre d'amortisseur (12) et la chambre d'équilibrage (24) étant agencée axialement à distance du piston, et notamment axialement adjacente à ladite chambre d'amortisseur (12), et de préférence axialement à côté de la deuxième chambre (17).

3. Amortisseur (1) selon la revendication précédente, la soupape d'étranglement (13) étant reliée à la chambre d'équilibrage (24) via un premier clapet de refoulement (26) qui ne permet qu'un passage de l'agent amortisseur (11) de ladite soupape d'étranglement (13) dans ladite chambre d'équilibrage (24) et/ou ladite chambre d'équilibrage (24) étant reliée à la deuxième chambre (17) via un deuxième clapet de refoulement (27) qui ne permet qu'un passage dudit agent amortisseur (11) de ladite chambre d'équilibrage (24) dans ladite deuxième chambre (17).

4. Amortisseur (1) selon l'une quelconque des revendications précédentes, un rapport entre le diamètre extérieur de la tige de piston et le diamètre extérieur du piston (14) étant de 0,2 à 0,4 et/ou un rapport entre le diamètre extérieur de ladite tige de piston et le diamètre extérieur dudit piston (14) étant adapté à un rapport prédéfini entre un amortissement de base dans l'action de compression et un amortissement de base dans l'action d'extension.

5. Amortisseur (1) selon l'une quelconque des revendications précédentes, la source de champ magnétique comprenant une bobine électrique en position couchée, montée à l'extérieur de la première et de la deuxième chambre, et dont un axe de bobine est orienté à la transversale du sens de passage de l'agent amortisseur magnéto-rhéologique.

6. Amortisseur (1) selon la revendication précédente, au moins un système capteur (33) étant prévu, celui-ci étant notamment prévu pour identifier au moins une grandeur de réglage.

7. Amortisseur (1) selon la revendication précédente, le système capteur (33) étant prévu pour détecter une mesure d'une vitesse relative et notamment pour détecter une mesure d'une vitesse relative du piston (15) par rapport à la chambre d'amortisseur (12) et/ou un système capteur (33) étant prévu pour détecter un sens (34, 35) du mouvement relatif entre le piston (15) et la chambre d'amortisseur (12) et/ou un système capteur (33) étant prévu pour détecter une mesure d'un débattement (36) et/ou au moins d'une accélération.

8. Amortisseur (1) selon l'une quelconque des revendications précédentes, la première chambre (16) étant, dans l'utilisation prévue, agencée au-dessous de la deuxième chambre (17) et la soupape d'étranglement (13), elle, au-dessus de la chambre d'amortisseur (12), et la chambre d'équilibrage (24) étant prévue au-dessus de ladite soupape d'étranglement (13).

9. Amortisseur (1) selon l'une quelconque des revendications précédentes, au moins la chambre d'amortisseur et la soupape d'étranglement étant agencées dans un complexe de tubes, et un système formant insert (38) étant prévu entre ledit complexe de tubes (3) et ladite chambre d'amortisseur (12), le canal de reflux (18) étant prévu, au moins sur certaines portions, au niveau du système formant insert (38) et délimitant de préférence la section droite de passage (39) dudit canal de reflux (18), une densité moyenne dudit système formant insert (38) entre le complexe de tubes (3, 4) étant inférieure à une densité moyenne de l'agent amortisseur (11).

10. Amortisseur (1) selon la revendication précédente, un diamètre maximal (40) d'une section droite de passage (39) du canal de reflux (18) au système formant insert (38) étant inférieur à un diamètre (41) et notamment inférieur à un rayon du complexe de tubes (3).

11. Amortisseur (1) selon l'une quelconque des revendications précédentes, le flux de l'agent amortisseur magnéto-rhéologique au niveau de la soupape d'étranglement (13) variant via la source de champ magnétique et son état de fonctionnement pouvant être ensuite maintenu sans courant.

12. Amortisseur (1) selon l'une quelconque des revendications précédentes, l'autre soupape d'étranglement étant conçue en tant que vanne de descente (42) et, de préférence, l'autre source de champ magnétique (43) comprenant une bobine électrique (44) et/ou un aimant rémanent (45) et/ou au moins un aimant permanent (46), et notamment ledit aimant permanent (46) étant au moins mobile entre une position normale et une position de descente (47) .

13. Amortisseur (1) selon l'une quelconque des revendications précédentes, pour une prothèse.

14. Amortisseur (1) selon l'une quelconque des revendications précédentes, pour un deux-roues à moteur.

15. Amortisseur (1) selon l'une quelconque des revendications précédentes, pour un siège d'un véhicule tel que, notamment, d'un véhicule particulier, camion, bus ou d'un autre véhicule utilitaire ou véhicule militaire, véhicule de transport de troupe, d'un blindé, d'un hélicoptère, d'un véhicule terrestre ou d'une machine de chantier.
